(19) Europäisches Patentamt
European Patent Office
Office européen des brevets

(11) **EP 4 528 256 A1**

(12) **EUROPEAN PATENT APPLICATION**
published in accordance with Art. 153(4) EPC

(43) Date of publication:
**26.03.2025 Bulletin 2025/13**

(21) Application number: **23807475.1**

(22) Date of filing: **08.05.2023**

(51) International Patent Classification (IPC):
**G01N 23/041** (2018.01)     **G01N 23/083** (2018.01)

(52) Cooperative Patent Classification (CPC):
**G01N 23/041; G01N 23/083**

(86) International application number:
**PCT/JP2023/017327**

(87) International publication number:
**WO 2023/223871 (23.11.2023 Gazette 2023/47)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **18.05.2022 JP 2022081885**

(71) Applicant: **SHIMADZU CORPORATION
Kyoto-shi, Kyoto 604-8511 (JP)**

(72) Inventors:
• **MORIMOTO, Naoki
Kyoto-shi, Kyoto 604-8511 (JP)**
• **KIMURA, Kenji
Kyoto-shi, Kyoto 604-8511 (JP)**
• **DOKI, Takahiro
Kyoto-shi, Kyoto 604-8511 (JP)**
• **NAGAI, ISHIKAWA, Lisa
Kyoto-shi, Kyoto 604-8511 (JP)**

(74) Representative: **Müller-Boré & Partner
Patentanwälte PartG mbB
Friedenheimer Brücke 21
80639 München (DE)**

(54) **X-RAY PHASE IMAGING DEVICE, X-RAY IMAGE PROCESSING DEVICE, X-RAY IMAGE PROCESSING METHOD, AND CORRECTION CURVE GENERATING METHOD**

(57)     A phase contrast X-ray imaging apparatus (100) according to this disclosure includes an X-ray source (1); an X-ray detector (2); a plurality of gratings; an image processor (7a) for generating a first dark field image (35a); a storage (8) for storing a plurality of correction curves (20a) generated at positions in a direction perpendicular to a grating direction; and a controller (7b) for correcting the first dark field image by using the correction curves corresponding to the positions, wherein each of the plurality of correction curves represents an assignment relation between values that relate to X-ray absorption of a subject (90) and values that relate to X-ray scattering of the subject at corresponding one of the positions.

FIG.1

EP 4 528 256 A1

## Description

Technical Field

The present invention relates to a phase contrast X-ray imaging apparatus, an X-ray image processing apparatus, an X-ray image processing method and a correction curve generation method, and in particular to a phase contrast X-ray imaging apparatus, an X-ray image processing apparatus, an X-ray image processing method and a correction curve generation method using a plurality of gratings.

Background Art

Phase contrast X-ray imaging apparatuses using a plurality of gratings for capturing images are known in the art. Such a phase contrast X-ray imaging apparatus is disclosed in Japanese Patent Laid-Open Publication No. JP 2021-194389, for example.

Japanese Patent Laid-Open Publication No. JP 2021-194389 discloses a radiography system including a radiation generator, a radiation detector, a plurality of gratings arranged between the radiation generator and the radiation detector, and an image processing apparatus for generating a small angle scattering image based on signals detected by the radiation detector.

Prior Art

Patent Document

Patent Document 1: Japanese Patent Laid-Open Publication No. JP 2021-194389

Summary of the Invention

Problems to be Solved by the Invention

Here, although not stated in Japanese Patent Laid-Open Publication No. JP 2021-194389, it is known that, when radiation (X-rays) passes through an object, a spectrum of X-rays changes. A hardening degree of the spectrum of X-rays varies in accordance with a thickness of the object through which the X-rays pass (a path length of X-rays). The change of the spectrum of X-rays also occurs when X-rays pass through the grating.

In radiography systems (phase contrast X-ray imaging apparatuses) such as the radiography system disclosed in Japanese Patent Laid-Open Publication No. JP 2021-194389, X-rays that radiate from a radiation generator (an X-ray source) radiate in a so-called cone-beam form. In this case, an incident angle of X-rays in a peripheral part of a grating is larger than a central part of the grating. If incident angles of X-rays with respect to the grating are different, path lengths of the X-rays passing through the grating correspondingly changes so that change degrees of spectra of X-rays become different. For this reason, difference between change degrees of spectra of X-rays causes uneven noise in a small angle scattering image (dark field image). In this case, there is a problem that image quality of the dark field image decreases due to noise caused by X-rays incident on the grating in an inclined direction.

The present invention is intended to solve the above problem, and one object of the present invention is to provide a phase contrast X-ray imaging apparatus, an X-ray image processing apparatus, an X-ray image processing method and a correction curve generation method capable of reducing deterioration of image quality of a dark field image even in a case in which X-rays are incident on a grating in an inclined direction.

Means for Solving the Problems

In order to attain the aforementioned object, the present inventors have diligently studied uneven noise, and as a result have found that uneven noise appears in a dark field image due to change of a scattering degree of X-rays caused by interaction between change of a spectrum of X-rays appearing in transmission of the X-rays through a subject and change of a spectrum of X-rays caused by difference between incident angles of the X-rays on a grating. That is, a phase contrast X-ray imaging apparatus according to a first aspect of the present invention includes an X-ray source; an X-ray detector for detecting the X-rays for irradiation from the X-ray source; a plurality of gratings arranged between the X-ray source and the X-ray detector; an image processor for generating a phase contrast X-ray image including a first dark field image, which is a dark field image of a subject, based on an intensity distribution of the X-rays detected by the X-ray detector; a storage for storing a plurality of correction curves generated at positions in a direction perpendicular to a grating direction in which the plurality of gratings extend; and a controller for correcting the first dark field image by using the correction curves corresponding to the positions, wherein each of the plurality of correction curves represents an assignment relation between values that relate to X-ray absorption of the subject and values that relate to X-ray scattering of the subject at corresponding one of the positions.

An X-ray image processing apparatus according to a second aspect of the present invention includes an image acquirer for acquiring a phase contrast X-ray image including a first dark field image, which is a dark field image of a subject; a storage for storing a plurality of correction curves generated at positions in a direction perpendicular to a grating direction in which a plurality of gratings extend; and a controller for correcting the first dark field image by using the correction curves corresponding to the positions, wherein each of the plurality of correction curves represents an assignment relation between values that relate to X-ray absorption of the subject and values that relate to X-ray scattering of the subject at corresponding one of the positions.

An X-ray image processing method according to a third aspect of the present invention includes acquiring a phase contrast X-ray image including a first dark field image, which is a dark field image of a subject; storing a plurality of correction curves generated at positions in a direction perpendicular to a grating direction in which a plurality of gratings extend; and correcting the first dark field image by using the correction curves corresponding to the positions, wherein each of the plurality of correction curves represents an assignment relation between values that relate to X-ray absorption of the subject and values that relate to X-ray scattering of the subject at corresponding one of the positions.

A correction curve generation method according to a fourth aspect of the present invention includes acquiring a second dark field image, which is a dark field image of a correction phantom, by capturing an image of the correction phantom by using a phase contrast X-ray imaging apparatus including a plurality of gratings; acquiring a second absorption image, which is an absorption image of the correction phantom; and generating the plurality of correction curves at positions in the direction perpendicular to the grating direction in which the plurality of gratings extend by using the second dark field image and the second absorption image.

Effect of the Invention

In the phase contrast X-ray imaging apparatus according to the first aspect, the X-ray image processing apparatus according to the second aspect, and the X-ray image processing method according to the third aspect, a plurality of correction curves corresponding to the positions in a direction perpendicular to the grating direction is stored. Because each of the plurality of correction curves represents an assignment relation between values that relate to X-ray absorption of the subject and values that relate to X-ray scattering of the subject at corresponding one of the positions, a change degree of a spectrum of X-rays depending on an incident angle of the X-rays at each position is taken into account. Also, the first dark field image can be accurately corrected by correcting the first dark field image, which is a dark field image of a subject, by using the correction curves corresponding to the positions, as compared with a case in which the first dark field image is corrected by using a single common correction curve. Consequently, it is possible to reduce deterioration of image quality of a first dark field image even in a case in which X-rays are incident on a grating in an inclined direction.

In the correction curve generation method according to the fourth aspect of the present invention, because the plurality of correction curves are generated by using the second dark field image and the second absorption image acquired by actually capturing the correction phantom, it is possible to generate the plurality of correction curves with a change degree of a spectrum of X-rays depending on an incident angle of the X-rays at each position being highly accurately being reflected.

Brief Description of the Drawings

FIG. 1 is a schematic diagram showing an entire configuration of a phase contrast X-ray imaging apparatus including an X-ray image processing apparatus according to one embodiment.

FIG. 2 is a schematic view showing a configuration of a grating position adjustment mechanism of the phase contrast X-ray imaging apparatus according to the one embodiment.

FIG. 3 is a schematic diagram illustrating a configuration of the X-ray image processing apparatus that generates phase contrast X-ray images according to the one embodiment.

FIG. 4 is a schematic view illustrating uneven noise appearing in a dark field image.

FIG. 5 is a graph illustrating how a pixel value varies depending on a position along a dashed line in FIG. 4 in the dark field image.

FIG. 6 is a schematic view illustrating difference between incident angles of X-rays on a grating.

FIG. 7 is a graph illustrating change of a spectrum of X-rays caused by the difference between incident angles of the X-rays on the grating.

FIG. 8 is a schematic view illustrating a shape and an arrangement of a correction phantom.

FIG. 9 is a schematic view illustrating thickness difference of the correction phantom.

FIGS. 10(A) and 10(B) are schematic diagrams illustrating a configuration for generating a correction curve.

FIG. 11 is a graph illustrating a correction-curve set including a plurality of correction curves.

FIG. 12 is a schematic diagram illustrating a configuration for generating different types of a plurality of correction-curve sets by using correction phantoms of different materials and different imaging conditions.

FIGS. 13(A) and 13(B) are schematic diagrams illustrating a configuration in which a subject image is corrected by a controller according to the one embodiment, and FIG. 13(C) is a graph illustrating the correction.

FIGS. 14(A), 14(C) and 14(E) are schematic diagrams illustrating difference of correction accuracy between a dark field image after correction in a comparative example and a dark field image after correction in the one embodiment, and FIGS. 14(B), 14(D) and 14(F) are graphs illustrating the difference of correction accuracy.

FIG. 15 is a flowchart illustrating processing of the X-ray image processing apparatus for generating correction curves according to the one embodiment.

FIG. 16 is a flowchart illustrating processing of the X-ray image processing apparatus for correcting a subject image according to the one embodiment.

FIG. 17 is a schematic view illustrating a correction phantom according to a first modified embodiment.

FIG. 18 is a schematic view illustrating a correction phantom according to a second modified embodiment.

FIGS. 19(A) and 19(B) are schematic views illustrating difference between energy distributions of X-rays depending on orientations of the grating.

Modes for Carrying Out the Invention

Embodiments embodying the present invention will be described with reference to the drawings.

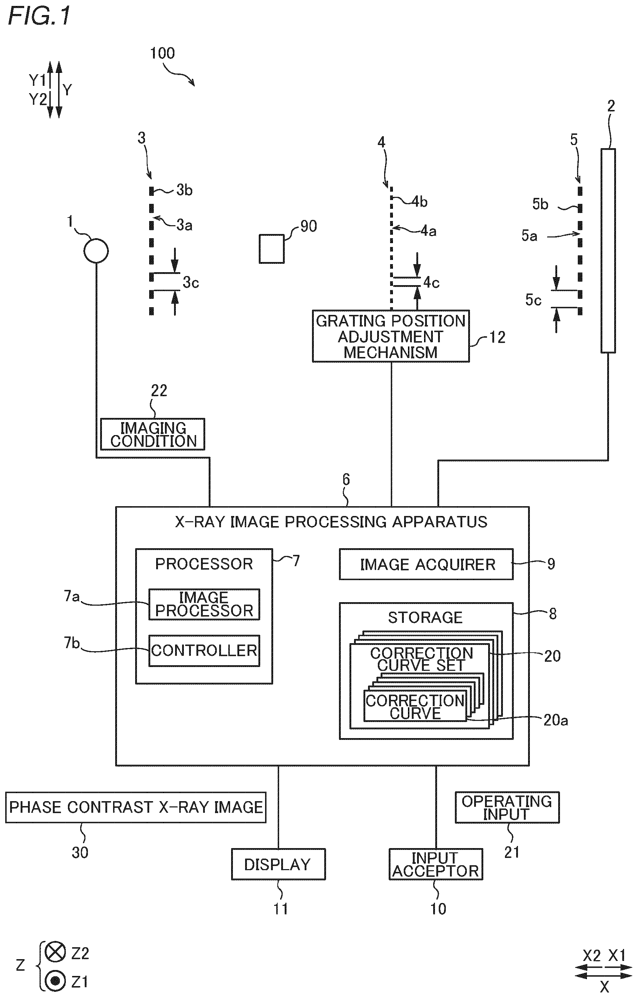

An entire configuration of a phase contrast X-ray imaging apparatus 100 including an X-ray image processing apparatus 6 according to one embodiment of the present invention is now described with reference to FIG. 1.

As shown in FIG. 1, the phase contrast X-ray imaging apparatus 100 is an apparatus for imaging an interior of a subject 90 by using the Talbot effect. The phase contrast X-ray imaging apparatus 100 includes an X-ray source 1, an X-ray detector 2, a plurality of gratings, and the X-ray image processing apparatus 6. In addition, the phase contrast X-ray imaging apparatus 100 includes an input acceptor 10, a display 11, and a grating position adjustment mechanism 12.

In this specification, an upward/downward direction is defined as a Z direction, and upward and downward directions are defined as Z1 and Z2 directions, respectively. Also, a direction between the X-ray source 1 and the X-ray detector 2 is defined as an X direction, and one direction is defined as an X1 direction while another direction is defined as an X2 direction in the X direction. In an illustration of FIG. 1, the X direction is a direction perpendicular to the Z direction. Also, a direction orthogonal to the Z direction and the X direction is defined as a Y direction, and one direction is defined as a Y1 direction while another direction is defined as a Y2 direction. The illustration of FIG. 1 is a plan view of the phase contrast X-ray imaging apparatus 100.

The X-ray source 1 is configured to irradiate the subject 90 with X-rays. Specifically, the X-ray source 1 is configured to generate X-rays when a high voltage is applied.

The X-ray detector 2 is configured to detect X-rays with which the subject is irradiated by the X-ray source 1. The X-ray detector 2 is configured to convert the detected X-rays into electrical signals. The X-ray detector 2 is a flat panel detector (FPD), for example. The X-ray detector 2 includes a plurality of converters (not shown), and a plurality of pixel electrodes (not shown) arranged on the plurality of converters. The plurality of converters and the plurality of pixel electrodes are aligned at a predetermined period (pixel pitch) in the Y and Z directions. The detection signals (image signals) of the X-ray detector 2 are transmitted to the X-ray image processing apparatus 6.

The plurality of gratings are arranged between the X-ray source 1 and the X-ray detector 2. In this embodiment, the plurality of gratings include a first grating 3, a second grating 4, and a third grating 5.

The first grating 3 is arranged between the X-ray source 1 and the second grating 4. The first grating 3 is irradiated with X-rays from the X-ray source 1. The first grating 3 has a plurality of slits 3a and X-ray absorption parts 3b, which are aligned at a predetermined cycle (pitch) 3c in the Z direction. The slits 3a and the X-ray absorption parts 3b are formed to linearly extend in the Y direction. Also, the slits 3a and the X-ray absorption parts 3b are formed to extend parallel to each other. The first grating 3 is configured to change X-rays into stripes of X-rays corresponding to positions of the slits 3a as a stripe-pattern light source after the X-rays pass through the slits 3a.

The second grating 4 is arranged between the first grating 3 and the third grating 5. The second grating 4 is irradiated with X-rays from the first grating 3. The second grating 4 has slits 4a and X-ray phase change parts 4b, which are arranged at a predetermined cycle (grating pitch) 4c in the Y direction. The slits 4a and the X-ray phase change parts 4b are formed to linearly extend in the Z direction. The second grating 4 is a so-called phase grating. The second grating 4 is provided to form a self-image when irradiated with X-rays from the X-ray source 1 (by using the Talbot effect). Here, the Talbot effect refers to formation of an image of a grating (self-image) having slits at a certain distance (Talbot distance) from the grating when coherent X-rays pass through the grating.

The third grating 5 is irradiated with X-rays from the second grating 4. The third grating 5 has a plurality of X-ray transmission parts 5a and X-ray absorption parts 5b, which are aligned at a predetermined cycle (grating pitch) 5c in the Y direction. The X-ray transmission parts 5a and the X-ray absorption parts 5b are formed to linearly extend in the Z direction. The third grating 5 is a so-called absorption grating. The third grating 5 is arranged between the second grating 4 and the X-ray detector 2, and is configured to interfere with the self-image formed by the second grating 4. To interfere the self-image with the third grating 5, the third grating 5 is position at the Talbot distance away from the second grating 4.

In this embodiment, the first grating 3, the second grating 4, and the third grating 5 are orientated to make their grating directions agree with the Z direction. The grating direction is a direction in which a grating pattern forming part extends. The grating pattern forming part in the first grating 3 is a part that is constructed of the slits 3a and the X-ray absorption parts 3b, and serves as a grating. The grating pattern forming part in the second grating 4 is constructed of the slits 4a and the X-ray phase change parts 4b. The grating pattern forming part in the third grating 5 is constructed of the X-ray transmission parts 5a and the X-ray absorption parts 5b.

The X-ray image processing apparatus 6 includes a processor 7, such as a CPU (Central Processing Unit), a GPU (Graphics Processing Unit) or an FPGA (Field-Programmable Gate Array) configured for image processing, a memory, such as a ROM (Read Only Memory) and a RAM (Random Access Memory), a storage 8, and an image acquirer 9, for example. The X-ray image processing apparatus 6 may include a circuitry instead of the processor 7.

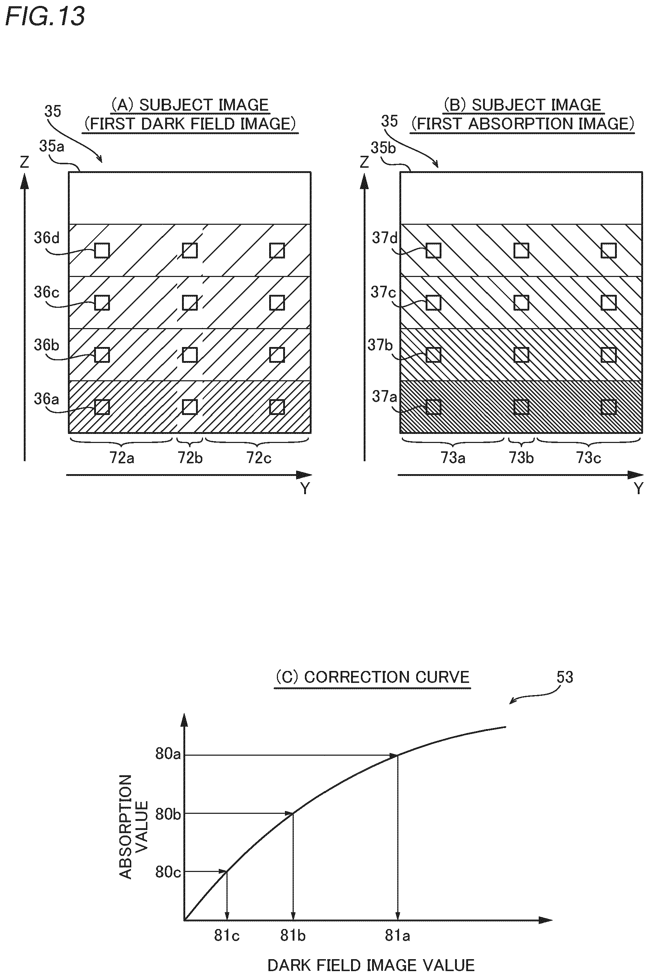

The processor 7 includes an image processor 7a and a controller 7b. The image processor 7a is configured to generate a phase contrast X-ray image 30 based on an intensity distribution of X-rays detected by the X-ray detector 2. The image processor 7a is constructed of software as a function block realized by executing the various programs by the processor 7. The image processor 7a may be constructed of hardware as a dedicated processing circuit. In this embodiment, the phase contrast X-ray image 30 includes a first dark field image 35a (see FIG. 13(A)). In this embodiment, the phase contrast X-ray image 30 includes a first absorption image 35b (see FIG. 13(B)). A configuration of the image processor 7a that generates the phase contrast X-ray images 30 will be described in detail later.

The controller 7b is configured to control the X-ray source 1, the grating position adjustment mechanism 12, and the like. The controller 7b is constructed of software as a function block realized by executing the various programs by the processor 7. The controller 7b may be constructed of hardware as a dedicated processing circuit.

The storage 8 is configured to store a plurality types of correction curve sets 20, which will be described later. Each correction curve set 20 includes a plurality types of correction curves 20a, which will be discussed later. Also, the storage 8 is configured to store the phase contrast X-ray images 30 generated by the image processor 7a, and various programs to be executed by the processor 7. The storage 8 includes a storage device such as an HDD (Hard Disk Drive) or a nonvolatile memory e.g., an SSD (Solid State Drive), for example.

The image acquirer 9 is configured to acquire the phase contrast X-ray image 30 including the first dark field image 35a. The image acquirer 9 is an input/output interface, for example.

The input acceptor 10 is configured to accept an operating input 21 from a user. The input acceptor 10 includes an input device such as a keyboard, computer mouse, etc. for example.

The display 11 displays the phase contrast X-ray image 30 generated by the image processor 7a. For example, the display 11 includes a display device such as an LCD monitor, an organic EL (Electro Luminescence) monitor, or the like.

The grating position adjustment mechanism 12 is configured to be able to move the second grating 4 in the X, Y and Z directions, in a rotation direction Rz about an axis extending in the Z direction, a rotation direction Rx about an axis extending in the X direction, and a rotation direction Ry about an axis extending in the Y direction. A detailed configuration of the grating position adjustment mechanism 12 will be described later.

(Grating Position Adjustment Mechanism)

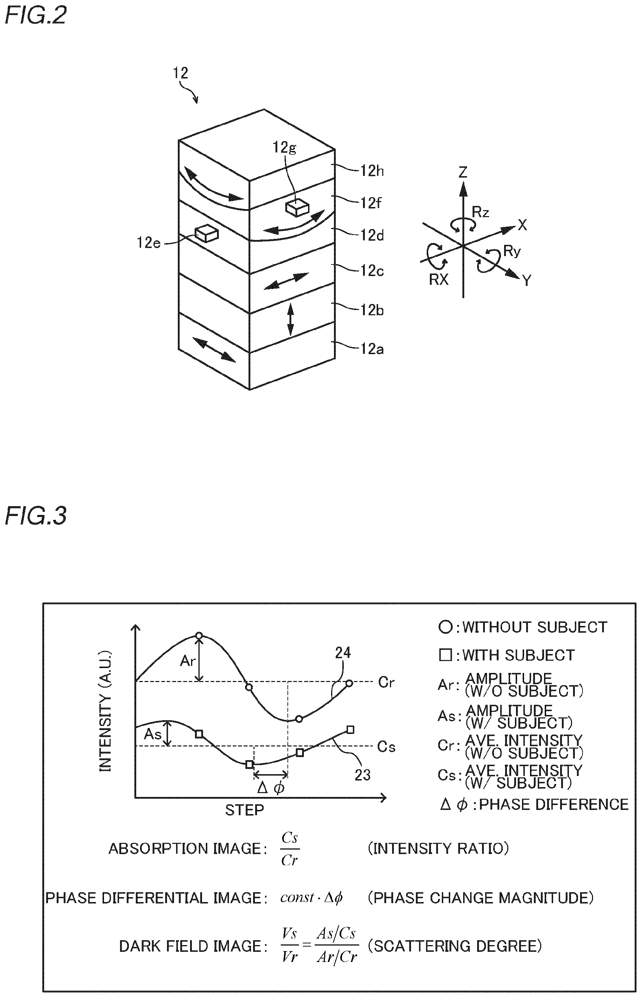

As shown in FIG. 2, the grating position adjustment mechanism 12 includes a Y-directional linear motion mechanism 12a, a Z-directional linear motion mechanism 12b, and an X-directional linear motion mechanism 12c, a linear motion mechanism connection part 12d, a stage support driver 12e, a stage support 12f, a stage driver 12g, and a stage 12h.

The Y-directional linear motion mechanism 12a, the Z-directional linear motion mechanism 12b, and the X-directional linear motion mechanism 12c are configured to move in the Y direction, the Z direction and the X direction, respectively. The Y-directional linear motion mechanism 12a, the Z-directional linear motion mechanism 12b, and the X-directional linear motion mechanism 12c include a driver such as a stepping motor, a drive force transmitter for transmitting a drive force from the driver, and a mover to be moved by the drive force transmitted by the drive force transmitter, for example. The grating position adjustment mechanism 12 is configured to move the second grating 4 by operating Y-directional linear motion mechanism 12a, the Z-directional linear motion mechanism 12b, and the X-directional linear motion mechanism 12c in the Y direction, the Z direction and the X direction, respectively.

The stage support 12f supports the stage 12h for mounting the second grating 4 from a lower side (Z2-direction side) of FIG. 2. The stage driver 12g is configured to be able to move the stage 12h back and forth in the Y direction. The stage 12h has a bottom surface formed in a convex surface bulging toward the stage support 12f, and is configured to rotate about the axis extending in the X direction (to rotate in the Rx direction) when moved in the Y direction. Also, the stage support driver 12e is configured be able to move the stage support 12f back and forth in the X direction. Also, the stage support 12f has a bottom surface formed in a convex surface bulging toward the linear motion mechanism connection part 12d, and is configured to rotate about the axis extending in the Y direction (to rotate in the Ry direction) when moved in the X direction. Also, the linear motion mechanism connection part 12d is provided to the X-directional linear motion mechanism 12c to be able to rotate about the axis extending in the Z direction (to rotate in the Rz direction). According to the aforementioned configuration, the grating position adjustment mechanism 12 can move the second grating 4 in a series of steps (translationally move the second grating) in the X direction, the Y direction, and the Z direction.

(Configuration of Phase Contrast X-Ray Image Generation)

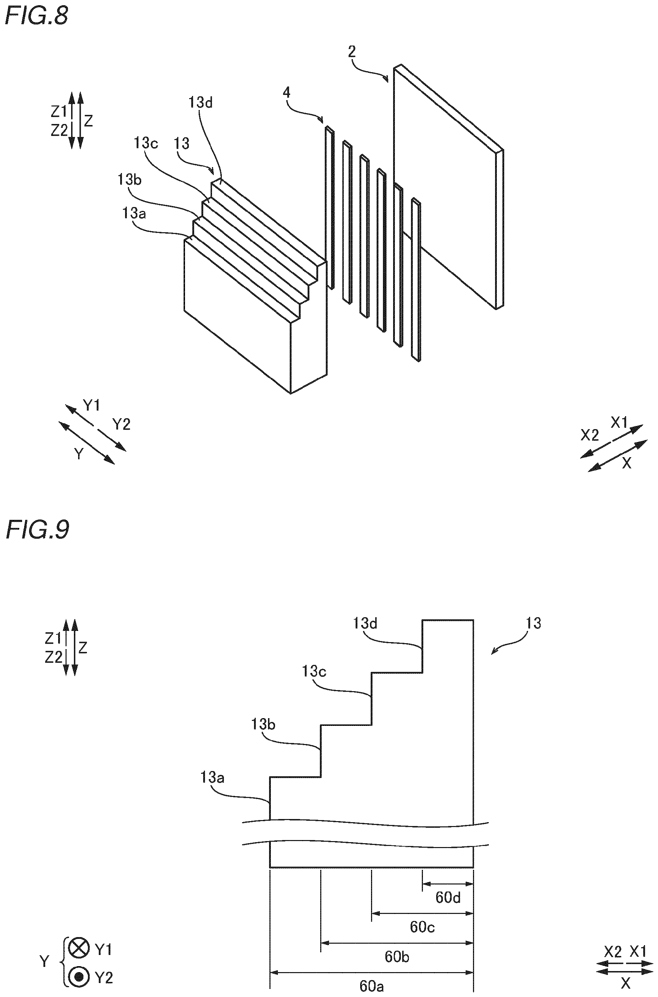

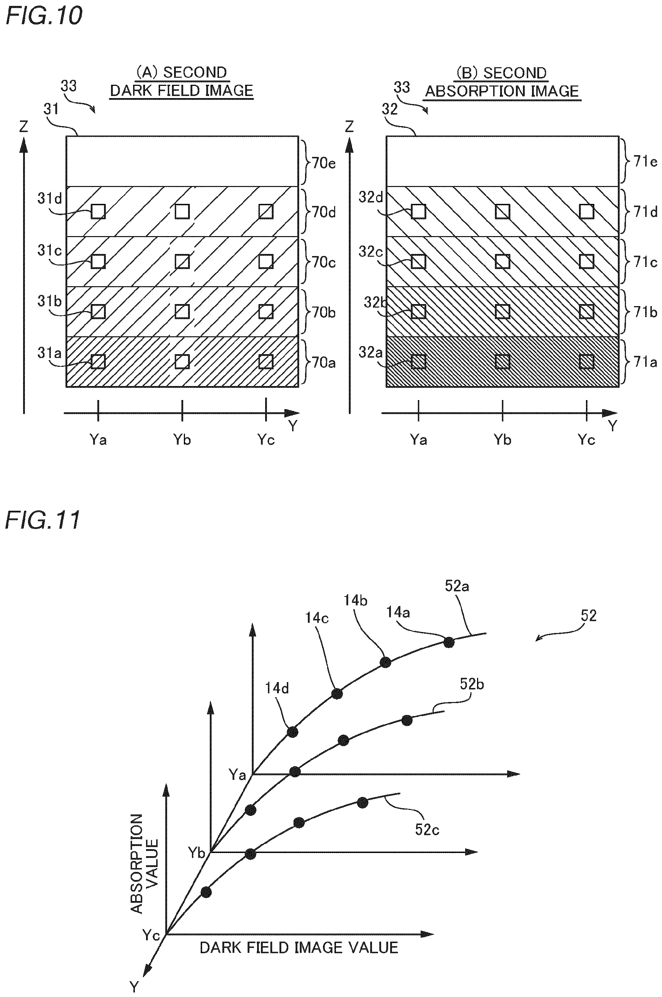

Processes executed by the image processor 7a (see FIG. 1) to generate the phase contrast X-ray image 30 (see FIG. 1) is now described with reference to FIG. 3. The image processor 7a generates the phase contrast X-ray image 30 using an intensity signal curve 23 and an intensity signal curve 24 acquired based on an intensity distribution of X-rays detected by the X-ray detector 2 (see FIG. 1) while translationally moving the second grating 4 by using the grating position adjustment mechanism 12 (see FIG. 1). The phase contrast X-ray image 30 includes a first absorption image 35b (see FIG. 13(B)), a second absorption image 32 (see FIG. 10(B)), the phase differential image, a first dark field image 35a (see FIG. 13(A)), and a second dark field image 31 (see FIG. 10(A)). The intensity signal curve 23 is a curve representing a distribution of intensity of X-rays acquired by capturing an image with the subject 90 (see FIG. 1) being placed. The intensity signal curve 24 is a curve representing a distribution of intensity of X-rays acquired by capturing an image without the subject 90 being placed. In this embodiment, description of the phase differential image is omitted. The first dark field image 35a and the first absorption image 35b are a dark field image of the subject 90 captured and an absorption image of the subject 90 captured, respectively. The second dark field image 31 and the second absorption image 32 are a dark field image of a correction phantom 13 (see FIG. 8) captured and an absorption image of the correction phantom 13 captured, respectively.

As shown in FIG. 3, the second absorption image 32 can be generated based on a ratio of average intensity Cs of X-rays when the image is captured with the subject 90 being placed to average intensity Cr of X-rays when the image is captured without the subject 90 being placed. Also, the second dark field image 31 can be generated based on a ratio of Visibility (Vr) of X-rays when the image is captured without the subject 90 being placed to Visibility (Vs) of X-rays when the image is captured with the subject 90 being placed. Vr can be calculated by a ratio of an amplitude Ar of the intensity signal curve 24 to the average intensity Cr. Vs can be calculated by a ratio of an amplitude As of the intensity signal curve 23 to the average intensity Cs.

(Noise Appearing in Dark Field Image)

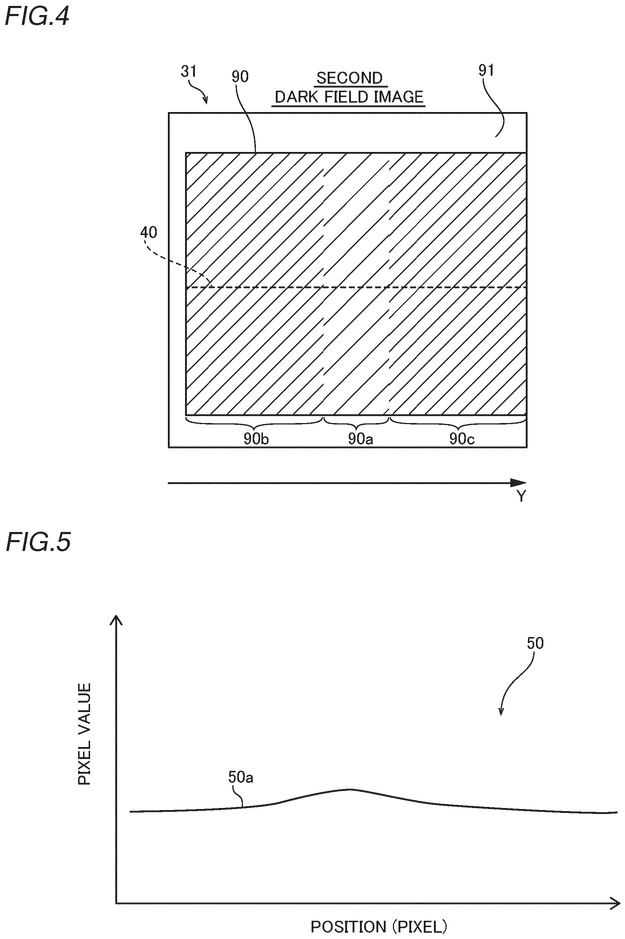

The following description describes uneven noise that appears in the second dark field image 31 (see FIG. 4) with reference to FIGS. 4 to 7. Here, difference of pixel values is represented by different hatching patterns in the second dark field image 31 shown in FIG. 4. Specifically, the thinner the hatching pattern (the wider the distance between hatching lines), the larger the pixel value. Here, illustrations of FIGS. 4 to 7 represent the uneven noise that appears in the second dark field image 31 generated by the phase contrast X-ray imaging apparatus 100 according to this embodiment, and is to be reduced by correction processing, which will be described later.

The illustration of FIG. 4 is a second dark field image 31 when an image of a flat plate-like aluminum material as the subject 90 is captured. The plate-like aluminum material does not normally induce X-ray scattering so that the plate-like aluminum material will not be imaged in the second dark field image 31. However, when an image of the plate-like aluminum material is captured, the subject 90 that includes pixel values different from a background 91 is imaged in the second dark field image 31 as shown in FIG. 4. This can be conceived due to change (hardening) of a spectrum of X-rays when the X-rays pass through the subject 90. Here, hardening of a spectrum of X-rays means that a central energy of the spectrum of the X-rays shifts to a higher energy side.

Also, as shown in FIG. 4, an area 90a in a central part in the Y direction of the subject 90 includes a pixel value higher than an area 90b and an area 90c on both sides in the second dark field image 31.

FIG. 5 is a graph 50 of pixel values plotted along a line 40 extending in the Y direction in the second dark field image 31 shown in FIG. 4. A vertical axis indicates the pixel value, and a horizontal axis indicates positions (pixels) in the graph 50.

As shown by a curve 50a in the graph 50, it is confirmed that the pixel value of area 90a (see FIG. 4) in the central part of the second dark field image 31 (see FIG. 4) is higher than the pixel values of the area 90b (see FIG. 4) and the area 90c (see FIG. 4) on both sides.

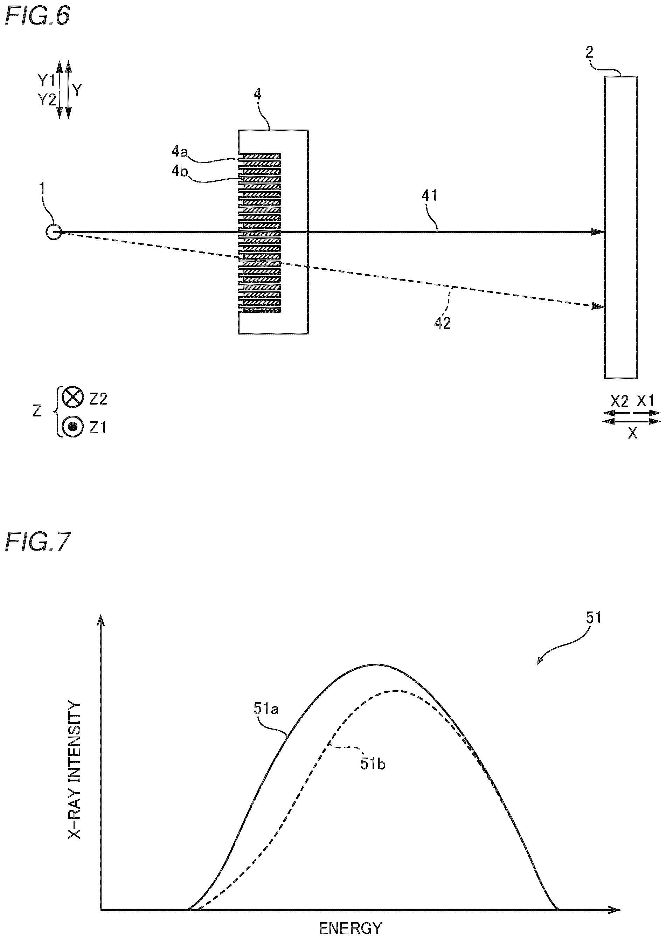

Here, X-rays for irradiation from the X-ray source 1 (see FIG. 6) are so-called cone-beam X-rays. Accordingly, the X-rays are incident on the plurality of gratings in an inclined direction. Because a hardening degree of a spectrum of X-rays changes due to difference between incident angles of the X-rays on the plurality of gratings, the pixel values of the area 90a in the central part of the second dark field image 31 becomes greater than the pixel values of the area 90b and the area 90c on the both sides.

An arrow 41 in FIG. 6 indicates X-rays that travel along an optical axis of the X-rays and with which the second grating 4 is irradiated. A dashed arrow 42 indicates X-ray that is incident from on the grating in an inclined direction. Here, in an illustration of FIG. 6, the second grating 4 is only shown in the plurality of gratings for ease of illustration. A spectrum of X-rays when the X-rays indicated by the arrow 41 pass through the second grating 4 and are detected by the X-ray detector 2 becomes different from a spectrum of X-rays when the X-rays indicated by the arrow 42 pass through the second grating 4 and are detected by the X-ray detector 2, because path lengths of the X-rays passing through the second grating 4 are different due to difference between incident angles of the X-rays on the second grating 4.

A graph 51 shown in FIG. 7 represents a spectrum of X-rays that pass through the second grating 4 (see FIG. 6). A vertical axis indicates X-ray intensity, and a horizontal axis indicates X-ray energy in the graph 51.

A curve 51a shown in the graph 51 represents a spectrum of the X-rays indicated by the arrow 41 in FIG. 6. A dashed curve 51b in the graph 51 represents a spectrum of the X-rays indicated by the dashed arrow 42 in FIG. 6.

In a case in which X-rays are incident on the second grating 4 in an inclined direction, a path length of the X-rays indicated by the arrow 42 for passing through the second grating 4 is greater than a path length of the X-rays indicated by the arrow 41. Because the path lengths are different, the spectrum of the X-rays represented by the curve 51b after passage through the second grating 4 becomes harder than the spectrum of the X-rays represented by the curve 51a. Because a hardening degree of a spectrum of X-rays changes in accordance with an incident angle of the X-rays on the second grating 4, uneven noise appears in a direction (Y direction) perpendicular to the grating direction (Z direction) in the second dark field image 31. If uneven noise appears in the second dark field image 31, a profile of pixel values has a convex shape having a center part bulging upward as shown by the curve 50a in the graph 50 shown in FIG. 5.

In a case in which an image of the subject 90 is captured by using an X-ray image processing apparatus having a plurality of gratings as in the phase contrast X-ray imaging apparatus 100 according to this embodiment, a change of a spectrum of X-rays due to the subject 90 and a change of the spectrum of the X-rays due to difference between incident angles of the X-rays on the second grating 4 occur. For this reason, as shown in FIG. 4, uneven noise appears in the second dark field image 31, and image quality of the second dark field image 31 deteriorates. The uneven noise appearing in the second dark field image 31 is caused by a combination of the change of the spectrum of the X-rays due to the subject 90 and the change of the spectrum of the X-rays due to the difference between incident angles of the X-rays on the second grating 4.

To address this, in this embodiment, the controller 7b is configured to correct the first dark field image 35a based on a plurality of correction curves 20a, which are generated at positions in the direction (Y direction) perpendicular to the grating direction (Z direction), and represents assignment relations between values that relate to X-ray absorption of the subject 90 and values that relate to X-ray scattering (scattering degree) of the subject at each of the positions. Each of the plurality of correction curves 20a are generated based on a correction image 33. The correction image 33 is an image of the correction phantom 13 captured.

(Correction Phantom)

The correction phantom 13 used to generate the plurality of correction curves 20a is first described with reference to FIGS. 8 and 9. Here, the gratings are arranged with their grating directions are the same direction as each other, in an illustration of FIG. 8, only the second grating 4 is shown for ease of illustration.

As shown in FIG. 8, the correction phantom 13 has a structure whose X-ray absorption degree changes in a direction of the grating extension (Z direction). The correction phantom 13 is formed to change its thickness in an X-ray irradiation direction (X direction) in the Z direction. Specifically, the correction phantom 13 has a stepped shape in which its thickness in the X-ray irradiation direction changes in steps in the Z direction. The correction phantom 13 is integrally formed of a homogeneous material.

The correction phantom 13 has a plurality of steps. Specifically, the correction phantom 13 has a first step 13a to a fourth step 13d.

As shown in FIG. 9, in thicknesses of the steps, a thickness 60a of the first step 13a is the largest, and a thickness 60d of the fourth step 13d is a the smallest.

A thickness per step of the correction phantom 13 is constant. In other words, with reference to the thickness 60d of the fourth step 13d, the thickness 60c of the third step 13c is twice the thickness 60d. Similarly, the thickness 60b of the second step 13b is triple the thickness 60d. Also, the thickness 60a of the first step 13a is four times the thickness 60d. Note that a thickness per step of the correction phantom 13 may not be constant.

(Correction Image)

Correction images 33 as shown in FIGS. 10(A) and 10(B) are obtained by capturing an image of the correction phantom 13.

FIG. 10(A) is a schematic diagram showing the second dark field image 31, which is a dark field image of the correction phantom 13, in the correction images 33. Also, FIG. 10(B) is a schematic diagram showing the second absorption image 32, which is an absorption image of the correction phantom 13, in the correction images 33. Here, difference of pixel values is represented by different hatching patterns in FIGS. 10(A) and 10(B). Specifically, the denser the hatching pattern (the narrower the distance between hatching lines), the larger the pixel value in FIGS. 10(A) and 10(B).

In the second dark field image 31 shown in FIG. 10(A), areas having different pixel values imaged in accordance with the thicknesses of the correction phantom 13. Specifically, a first area 70a is the area where the first step 13a of the correction phantom 13 is imaged. Also, a second area 70b is the area where the second step 13b of the correction phantom 13 is imaged. Also, a third area 70c is the area where the third step 13c of the correction phantom 13 is imaged. Also, a fourth area 70d is the area where the fourth step 13d of the correction phantom 13 is imaged. Also, a fifth area 70e is the area where no correction phantom 13 is imaged (background).

In the second absorption image 32 shown in FIG. 10(B), areas having different pixel values depending on the thicknesses of the correction phantom 13 are imaged. Specifically, a first area 71a is the area where the first step 13a of the correction phantom 13 is imaged. Also, a second area 71b is the area where the second step 13b of the correction phantom 13 is imaged. Also, a third area 71c is the area where the third step 13c of the correction phantom 13 is imaged. Also, a fourth area 71d is the area where the fourth step 13d of the correction phantom 13 is imaged. Also, a fifth area 71e is the area where no correction phantom 13 is imaged (background).

As shown in FIGS. 10(A) and 10(B), uneven noise due to hardening of a spectrum of X-rays after passage through the subject 90, and hardening of a spectrum of the X-rays due to difference between incident angles of the X-rays on the plurality of gratings appears only in the second dark field image 31.

(Correction Curve)

A configuration of the controller 7b that generates the plurality of correction curves 20a is now described with reference to FIGS. 10 to 13.

In this embodiment, the plurality of correction curves 20a are generated based on pixel values in the second absorption image 32 and pixel values in the second dark field image 31 of the areas that have different thicknesses from each other of the correction phantom 13 captured in the correction image 33 with the correction phantom 13 being orientated to change its thickness in the grating direction (Z direction).

In other words, the controller 7b acquires an assignment relation between the pixel values of the second dark field image 31 (values that relate to X-ray scattering and are specified based on the second dark field image 31), and pixel values of the second absorption image 32 (values that relate to X-ray absorption and are specified based on the second absorption image 32), and generates the plurality of correction curves 20a. Accordingly, because the correction phantom 13 is formed of a homogeneous material, in a case in which a thickness of the correction phantom 13 is fixed, change of the pixel values of the second dark field image 31 with respect to the pixel values of the second absorption image 32 becomes constant. That is, a factor of the change of the pixel values of the second dark field image 31 with respect to the pixel values of the second absorption image 32 is limited to the difference between the thicknesses of the correction phantom 13. As a result, the correction curves 20a corresponding to the difference between the thicknesses of the subject 90 can be easily generated by generating the correction curves 20a by using the pixel values in the second absorption image 32 and the pixel values in the second dark field image 31 of the areas of the correction phantom 13 that have different thicknesses from each other depending on positions in the direction perpendicular to the grating direction.

FIG. 11 is a graph 52 showing the plurality of correction curves 20a. The graph 52 includes graphs aligned at their corresponding Y-directional positions in the correction image 33, and each of the graphs has a vertical axis indicating absorption values (exemplary values that relate to X-ray absorption) and a horizontal axis indicating dark field values (exemplary values that relate to X-ray scattering). Here, each of the dark field values and each of absorption values are acquired by the following Equations (1) and (2).

[Formula 1]

$$\alpha = \ln D \quad \cdots \quad (1)$$

$$\gamma = \ln T \quad \cdots \quad (2)$$

where $\alpha$ is the dark field value, D is a pixel value of the second dark field image 31, $\gamma$ is the absorption value, and T is a pixel value of the second absorption image 32. Here, the dark field and absorption values are expressed in natural logarithms.

The configuration of the controller 7b that generates the plurality of correction curves 20a is described by describing a configuration of the controller 7b that generates a curve 52a shown in the graph 52 as a representative.

In this embodiment, the controller 7b determines a position of a first plot 14a in the graph 52 based on the dark field value acquired in accordance with the pixel value of a first pixel 31a (see FIG. 10(A)) in the second dark field image 31 and the absorption value acquired in accordance with the pixel value of a first pixel 32a (see FIG. 10(B)) in the second absorption image 32. Also, the controller 7b determines a position of a second plot 14b in the graph 52 based on the dark field value acquired in accordance with the pixel value of a second pixel 31b (see FIG. 10(A)) in the second dark field image 31 and the absorption value acquired in accordance with the pixel value of a second pixel 32b (see FIG. 10(B)) in the second absorption image 32.

Also, the controller 7b determines a position of a third plot 14c in the graph 52 based on the dark field value acquired in accordance with the pixel value of a third pixel 31c (see FIG. 10(A)) in the second dark field image 31 and the absorption value acquired in accordance with the pixel value of a third pixel 32c (see FIG. 10(B)) in the second absorption image 32. Also, the controller 7b determines a position of a fourth plot 14d in the graph 52 based on the dark field value acquired in accordance with the pixel value of a fourth pixel 31d (see FIG. 10(A)) in the second dark field image 31 and the absorption value acquired in accordance with the pixel value of a fourth pixel 32d (see FIG. 10(B)) in the second absorption image 32.

Here, the correction image 33 includes random noise in some cases. In such a case, if a correction curve 20a is generated based on a pixel value of one pixel in the area of the correction phantom 13 that have the same thickness, accuracy of the correction curve 20a (approximation accuracy) may be reduced due to the random noise. To address this, in this embodiment, each of the plurality of correction curves 20a is generated based on pixel values of the second absorption image 32 and pixel values of the second dark field image 31 at different positions in the area that has the same thickness of the grating direction in the correction phantom 13 captured in the correction image 33. In other words, each of the plurality of correction curves 20a is generated based on representative values of pixel values of the second dark field image 31 and representative values of pixel values of the second absorption image 32. Each representative value is an average value of the pixel values in the same area that extends in the Y direction and has the same thickness, for example. That is, in a case in which the average value of the first pixel 31a is acquired, an average value of pixel values at a Y-directional position Ya in the first area 70a is acquired. As a result, the correction curve 20a can be generated by using representative pixel values of the second absorption image 32 each of which corresponds to a plurality of positions in each area that has the same thickness in the grating direction, and representative pixel values of the second dark field image 31 each of which corresponds to a plurality of positions in each area that has the same thickness in the grating direction, for example. Consequently, it is possible to suppress reduction of accuracy of the correction curve 20a that is caused by random noise appearing in the correction image 33 as compared with a configuration in which the correction curve 20a is generated based on pixel values of the second absorption image 32 and pixel values of the second dark field image 31 each of which corresponds to one position in each area in the correction phantom 13 that has the same thickness.

The controller 7b generates the correction curve 20a that is indicated by the curve 52a by approximation of the first plot 14a to the fourth plot 14d to a curve.

The controller 7b generates the correction curve 20a that is indicated by a curve 52b, and the correction curve 20a that is indicated by a curve 52c at Y-directional positions in the correction image 33 by using similar processing by changing one Y-directional position of the correction image 33 to another. That is, the controller 7b generates the correction curve 20a at a Y-directional position Ya, the correction curve 20a at Y-directional position Yb, and the correction curve 20a at Y-directional position Yc.

Here, change of pixel values of the second dark field image 31 in the Y direction is a gradual change, it is not necessary to generate the correction curve 20a at every pixel in the Y direction. For this reason, the controller 7b generates a predetermined number of correction curves 20a depending on Y-directional size of the correction image 33. In illustrations of FIGS. 10 and 11, the controller 7b generates three correction curves 20a. Note that the number of correction curves 20a to be generated may be any number other than three. For example, the number of correction curves 20a may be determined based on a profile of pixel values of the second dark field image 31 in the Y direction (based on the graph 50 shown in FIG. 5), or a correction curve 20a may be generated by each predetermined distance in the Y direction.

Here, transmittance of X-rays varies in accordance with X-ray absorptance and thickness of the subject 90. Accordingly, even in a case in which a thickness of the subject 90 and a thickness of the correction phantom 13 are equal to each other, if their X-ray absorptances are different, the difference between X-ray absorptances may reduce accuracy of the correction of the first dark field image 35a using the correction curves 20a. For this reason, it is preferable to change the correction phantom 13 to be used to generate the correction curves 20a depending on a material of the subject 90. Specifically, the correction phantom 13 is preferably changed depending on an elemental composition of the subject 90. For example, in a case in which the subject 90 is CFRP (Carbon Fiber Reinforced Plastics), it is preferable to use the correction phantom 13 that is formed by an acrylic material because the acrylic material has an X-ray absorptance close to CFRP and a homogeneous phantom of the acrylic material that has few defects and a small density variation can be easily obtained. Also, in a case in which the subject 90 is GFRP (Glass Fiber Reinforced Plastics), it is preferable to use the correction phantom 13 that is formed by an aluminum material because the aluminum material has an X-ray absorptance close to GFRP and a homogeneous phantom of the aluminum material that has few defects and a small density variation can be easily obtained.

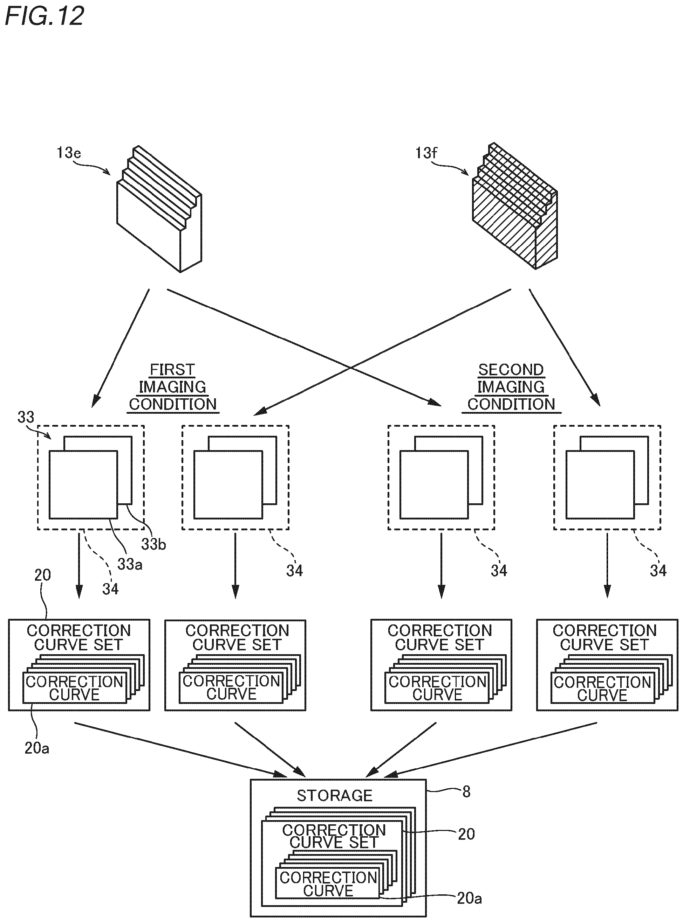

For this reason, in this embodiment, depending on materials of the different subjects 90, images of different correction phantoms 13 that have different X-ray absorptances are captured so that a plurality types of correction curve sets 20 are generated based on a plurality of correction image sets 34 (see FIG. 12) corresponding to different types of correction phantoms 13, and are included as the plurality of correction curves 20a.

Also, in a case in which setting of imaging conditions 22 for capturing the phase contrast X-ray image 30 is changed, change degrees of spectra of X-rays become different between before and after the changing of the imaging conditions 22. To address this, in this embodiment, the plurality of correction curves 20a include a plurality of correction curve sets 20, which are generated based on the correction images 33 captured under imaging conditions 22 different from each other, for the imaging conditions 22. Here, the imaging conditions 22 include a value of tube voltage applied to the X-ray source 1, and a rotation angle of the plurality of gratings.

A configuration of the controller 7b that generates the plurality types of correction curve sets 20 is now described with reference to FIG. 12.

In an illustration of FIG. 12, a first correction phantom 13e and a second correction phantom 13f are used to generate the plurality types of correction curve sets 20. The first correction phantom 13e is a phantom formed of an acrylic material, for example. Also, the second correction phantom 13f is a phantom formed of an aluminum material, for example.

Also, first and second imaging conditions are imaging conditions 22 that tube voltages applied to the X-ray source 1 are different from each other.

In the illustration of FIG. 12, the plurality of correction image sets 34 include the correction image 33 of the first correction phantom 13e that is captured under the first and second imaging conditions, and the correction image 33 of the second correction phantom 13f that is captured under the first and second imaging conditions so that the plurality of correction image sets include a total of the four correction images 33. Each of the four correction images 33 includes the dark field image 33a and the absorption image 33b.

The controller 7b generates the plurality of correction curve sets 20 using their respective four types of correction images 33. Subsequently, the controller 7b stores the plurality of correction curve sets 20 into the storage 8.

In this embodiment, the storage 8 stores the plurality of correction curves 20a associated with the imaging conditions 22. Also, the storage 8 stores the plurality of correction curves 20a associated with materials of the subjects 90. That is, in this embodiment, the storage 8 stores a plurality types of correction curve sets 20 associated with the imaging conditions 22 and X-ray absorptances. Specifically, the storage 8 stores the correction curve set 20 that is generated based on the correction image set 34 of the first correction phantom 13e that is captured under the first imaging condition, the correction curve set 20 that is generated based on the correction image set 34 of the second correction phantom 13f that is captured under the first imaging condition, the correction curve set 20 that is generated based on the correction image set 34 of the first correction phantom 13e that is captured under the second imaging condition, and the correction curve set 20 that is generated based on the correction image set 34 of the second correction phantom 13f that is captured under the second imaging condition.

Here, the correction curve 20a is a curve that assigns dark field values with absorption values. Accordingly, based on an absorption value acquired based on a value of a pixel of the second absorption image 32 and the correction curve 20a, a dark field value of the pixel that is assigned to the absorption value can be acquired.

(Correction of Dark Field Image)

A configuration of the controller 7b that corrects a subject image 35 is now described with reference to FIGS. 13(A) to 13(C). Here, the subject image 35 includes the first dark field image 35a shown in FIG. 13(A) and the first absorption image 35b shown in FIG. 13(B). The controller 7b corrects the first dark field image 35a in the subject image 35. A graph 53 shown in FIG. 13(C) shows the correction curve 20a that corresponds to a first area 72a (see FIG. 13(A)) in the plurality of correction curves 20a. A vertical axis indicates an absorption value, and a horizontal axis indicates a dark field value in the graph 53. In each of illustrations of FIGS. 13(A) and 13(B), an image including areas whose pixel values are different in the Z direction is shown for ease of understanding effects of the correction.

The controller 7b is configured to correct the first dark field image 35a by using the correction curves 20a corresponding to positions.

The controller 7b is configured to correct the first dark field image 35a based on the first absorption image 35b and the plurality of correction curves 20a. Because uneven noise causes a gradual change of pixel values in the Y direction, the controller 7b corrects the subject image 35 in the Z direction, for positions at a predetermined number of pixels from each other in the Y direction, by using corresponding one the correction curves 20a. In the illustration of FIG. 13(A), the controller 7b corrects the subject image 35 in the Z direction by using the correction curves 20a corresponding the first area 72a, the second area 72b, and the third area 72c.

As a representative of the configuration of the controller 7b that corrects the first dark field image 35a, correction of the subject image 35 (the first dark field image 35a) at the first area 72a is described. A first absorption value 80a of a first pixel 37a is acquired by using a pixel value of the first pixel 37a in a first area 73a of the first absorption image 35b corresponding to the first area 72a of the first dark field image 35a and the above Equation (2). Subsequently, the controller 7b acquires a first dark field value 81a based on the first absorption value 80a and the correction curve 20a.

Similarly, the controller 7b acquires a second absorption value 80b, a third absorption value 80c, and a fourth absorption value 80d based on a pixel value of a second pixel 37b, a pixel value of a third pixel 37c and a pixel value of a fourth pixel 37d in the first absorption image 35b, and the above Equation (2). Subsequently, the controller 7b acquires a second dark field value 81b, a third dark field value 81c, and a fourth dark field value 81d based on the second absorption value 80b, the third absorption value 80c and the fourth absorption value 80d, and the correction curve 20a. That is, the controller 7b is configured to access, for each of the positions in the first absorption image 35b, corresponding one of the correction curves 20a, and to specify values that relate to the X-ray scattering and are assigned to values that relate to the X-ray absorption in the first absorption image 35b.

The controller 7b acquires pseudo-signal components of pixels from dark field values of the first pixel 36a to the fourth pixel 36d of the first dark field image 35a by using the first dark field value 81a to the fourth dark field value 81d acquired, and the following Equation (3). Here, the pseudo-signal components are signal components of the first dark field image 35a that includes uneven noise.

[Formula 2]

$$D_f = \frac{1}{e^\alpha} \quad \cdots \quad (3)$$

where $D_f$ is a pixel value of each pseudo-signal component, $\alpha$ is the dark field value, and e is the base of the natural logarithm.

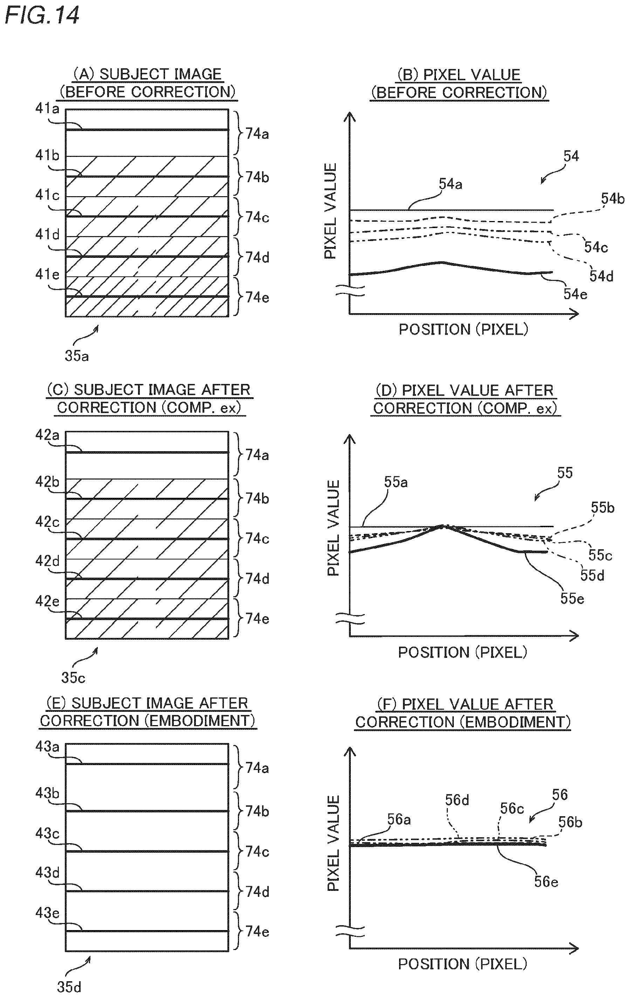

Subsequently, the controller 7b uses the pixel values of the pseudo-signal components of the pixels and the pixel value of the pixels to acquire pixel values of the pixels in a dark field image after correction 35d (see FIG. 14(E)) as shown in the following Equation (4).

[Formula 3]

$$D_{corr} = {D}\!/\!{D_f} \quad \cdots \quad (4)$$

where $D_{corr}$ is each pixel value of the dark field image after correction 35d.

The controller 7b corrects the first dark field image 35a, that is, corrects the second area 72b and the third area 72c of the first dark field image 35a by using the correction curves 20a corresponding to these areas, and the pixel values of the second area 73b and the third area 73c of the first absorption image 35b. In other words, the controller 7b is configured to correct the first dark field image 35a by using the values specified that relate to X-ray scattering.

Here, in this embodiment, the controller 7b is configured to select a plurality of correction curves 20a (see FIG. 1) in corresponding one set of the plurality types of correction curve sets 20 (see FIG. 1) depending on a material of the subject 90. Also, the controller 7b is configured to correct the first dark field image 35a (see FIG. 14(A)) by using the plurality of correction curves 20a selected. Specifically, the controller 7b is configured to select the correction curves 20a to be used to correct the first dark field image 35a based on the operating input 21 accepted by the input acceptor 10. For example, the controller 7b displays a combo box for selecting a type of the subject 90 on the display 11. The controller 7b acquires the correction curves 20a that are used to correct the first dark field image 35a in accordance with the material of the subject 90 input by an operator by selecting the material in the combo box. Accordingly, the first dark field image 35a is corrected by using the correction curves 20a corresponding to the operating input 21 of the operator. As a result, because the first dark field image 35a can be corrected by the correction curves 20a that are selected by the operator, it is possible to improve user convenience (usability).

The controller 7b is configured to select a plurality of correction curves 20a in accordance with the imaging condition 22. Also, the controller 7b is configured to correct the first dark field image 35a (see FIG. 14(A)) by using the plurality of correction curves 20a selected. Specifically, the controller 7b acquires a value of tube voltage applied to the X-ray source 1 as the imaging condition 22. The controller 7b acquires a plurality of correction curves 20a that are used to correct the first dark field image 35a from the plurality types of correction curve sets 20 in accordance with the value of tube voltage acquired.

In other words, the controller 7b acquires the plurality of correction curves 20a that are used to correct the first dark field image 35a (see FIG. 14(A)) from the plurality of correction curve sets 20 in accordance with the material of the subject 90 and the imaging condition 22.

(Effect of Correction of Dark Field Image)

The following description describes a dark field image after correction 35c, which is generated by correcting a subject image before correction (first dark field image 35a), in a comparative example, and a dark field image after correction 35d, which is generated by correcting the subject image before correction by using the correction curve set 20 corresponding to positions in the Y direction, in this embodiment with reference to FIGS. 14(A) to 14(F). The dark field image after correction 35c in the comparative example is corrected by using one type of correction curve set 20 independent of positions in the Y direction.

The first dark field image 35a shown in FIG. 14(A) is a dark field image before correction. A graph 54 of FIG. 14(B) shows changes of pixel values along a first line 41a to a fifth line 41e in a first area 74a to a fifth area 74e of the first dark field image before correction 35a, respectively.

An illustration of FIG. 14(C) shows the dark field image after correction 35c, which is generated by correcting the first dark field image before correction 35a by using one type of correction curve set 20 independent of positions in the Y direction, in a comparative example. Also, a graph 55 of FIG. 14(D) shows changes of pixel values along a first line 42a to a fifth line 42e in the first area 74a to the fifth area 74e of the first dark field image after correction 35c in the comparative example, respectively.

An illustration of FIG. 14(E) shows the dark field image after correction 35d, which is generated by correcting the first dark field image before correction 35a by using by using the correction curve set 20 corresponding to positions in the Y direction, in this embodiment. Also, a graph 56 of FIG. 14(F) shows changes of pixel values along a first line 43a to a fifth line 43e in the first area 74a to the fifth area 74e of the first dark field image after correction 35d in this embodiment, respectively.

A vertical axis indicates the pixel value, and a horizontal axis indicates the positions (pixels) in the graphs 54 to 56. A first curve 54a, a second curve 54b, a third curve 54c, a fourth curve 54d, and a fifth curve 54e shown in the graph 54 show changes of pixel values along the first line 41a, the second line 41b, the third line 41c, the fourth line 41d, and the fifth line 41e, respectively, in the areas of the dark field image before correction 35d.

Also, a first curve 55a, a second curve 55b, a third curve 5c, a fourth curve 55d, and a fifth curve 55e shown in the graph 55 show changes of pixel values along the first line 42a, the second line 42b, the third line 42c, the fourth line 42d, and the fifth line 42e, respectively, in the areas of the dark field image after correction 35c in the comparative example.

Also, a first curve 56a, a second curve 56b, a third curve 56c, a fourth curve 56d, and a fifth curve 56e shown in the graph 56 show changes of pixel values along the first line 43a, the second line 43b, the third line 43c, the fourth line 43d, and the fifth line 43e, respectively, in the areas of the dark field image after correction 35d in this embodiment.

In the first dark field image before correction 35a, as shown in the graph 54, the pixel values in the first area 74a to the fifth area 74e are different from each other. Also, in the second area 74b to the fifth area 74e, the pixel values in the central part of the image in the Y direction are high, and the pixel values on both sides are low as shown in the graph 54.

In the dark field image after correction 35c in the comparative example, as shown in the graph 55, difference between the pixel values in the first area 74a to the fifth area 74e becomes smaller than difference between the pixel values in the first dark field image before correction 35a. In addition, uneven noise is eliminated in the central part of the image. However, because the dark field image after correction 35c in the comparative example is corrected by using one type of correction curve set 20 independent of positions in the Y direction, the pixel values in the central part of the image are still high, and the pixel values on both sides are still low the second area 74b to the fifth area 74e as shown in the graph 55.

In the dark field image after correction 35d in this embodiment, as shown in the graph 56, difference between the pixel values in the first area 74a to the fifth area 74e substantially disappears. In addition, in the dark field image after correction 35d in this embodiment, difference between the pixel values in the central parts of the image and the pixel values in the both sides of the image substantially disappears in the second area 74b to the fifth area 74e. That is, in the dark field image after correction 35d in this embodiment, uneven noise due to interaction between both a change of a spectrum of X-rays caused by the subject 90 (see FIG. 1) and a change of the spectrum of X-rays caused by X-rays incident on the grating in an inclined direction is reduced.

(Generation Processing of Correction Curves and Correction Processing of Dark Field Image)

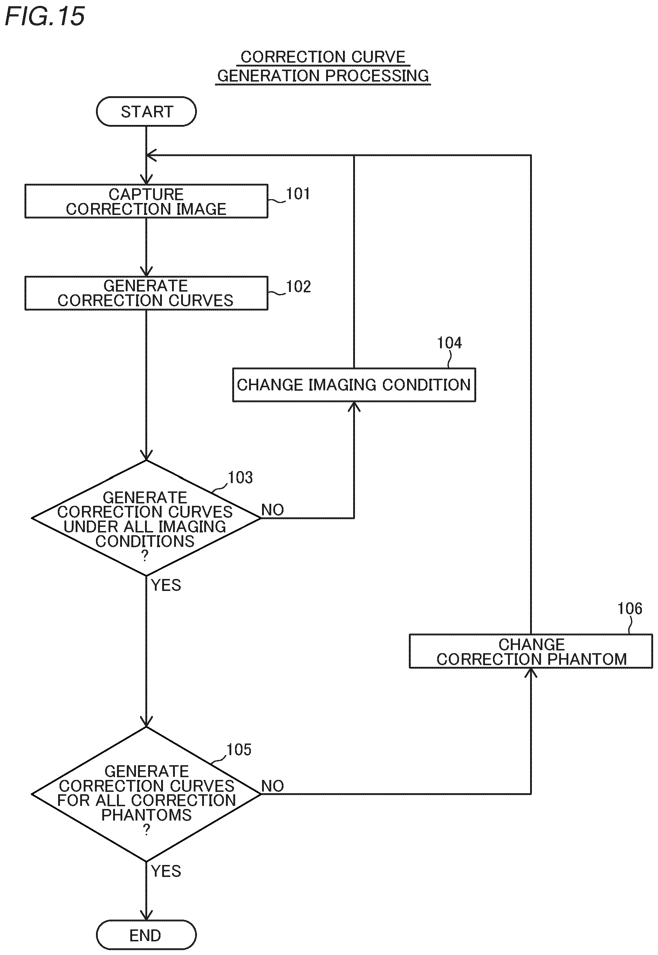

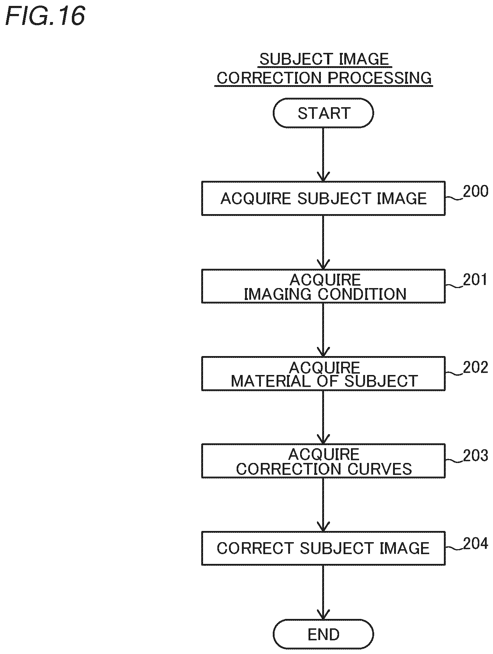

The following description describes a configuration of an X-ray image processing method by using the X-ray image processing apparatus 6 according to this embodiment with reference to FIGS. 15 and 16. In this embodiment, the X-ray image processing method includes processing for generating the correction curves 20a and processing for correcting the first dark field image 35a, which are main divided parts of the method.

The processing for generating the correction curves 20a by using the X-ray image processing apparatus 6 is first described with reference to FIG. 15.

In step 101, the controller 7b captures the correction image 33 including the second dark field image 31 (see FIG. 10(A)) and the second absorption image 32 (see FIG. 10(B)) of the correction phantom 13 captured by controlling the X-ray source 1.

The controller 7b captures the correction image 33 with the correction phantom 13 being orientated to make a direction in which a thickness of the correction phantom 13 changes agree with the grating direction (Z direction).

In step 102, the controller 7b generates the correction curves 20a based on the correction image 33.

Here, in step 102, the controller 7b generates the correction curves 20a based on pixel values in the second absorption image 32 and pixel values in the second dark field image 31 of the areas that have different thicknesses from each other of the correction phantom 13 captured in the correction image 33. Specifically, the controller 7b generates plurality of correction curves 20a based on pixel values in the second absorption image 32 and pixel values in the second dark field image 31 at different positions in the direction (Y direction) perpendicular to the grating direction (Z direction). More specifically, the controller 7b generates each of the plurality of correction curves 20a based on pixel values of the second absorption image 32 and pixel values of the second dark field image 31 at different positions in the area that has the same thickness of the grating direction in the correction phantom 13 captured in the correction image 33. Also, the controller 7b stores plurality of correction curves 20a generated into the storage 8.

In step 103, the controller 7b determines whether the plurality of correction curves 20a are generated under all of the predetermined imaging conditions 22. If the plurality of correction curves 20a are not generated under all of the predetermined imaging conditions 22, the procedure goes to step 104. If the plurality of correction curves 20a are generated under all of the predetermined imaging conditions 22, the procedure goes to step 105.

In step 104, controller 7b changes one of the imaging conditions 22 to another. Subsequently, the procedure goes to step 101.

If the procedure goes from step 103 to step 105, in step 105, the controller 7b determines whether the plurality of correction curves 20a are generated in all of the plurality of correction phantoms 13 (see FIG. 8). If the plurality of correction curves 20a are not generated in all of the plurality of correction phantoms 13, the procedure goes to step 106. If the plurality of correction curves 20a are generated in all of the plurality of correction phantoms 13, the procedure ends.

In step 106, controller 7b changes one of the correction phantoms 13 to another. For example, the controller 7b urges operators to change the correction phantom 13 by displaying a message to change the correction phantom 13 on the display 11. If the correction phantom 13 is changed, the procedure goes to step 101.

As described above, each of the plurality of correction curve sets 20 is generated under corresponding one of the imaging conditions 22 in steps for capturing an image of the correction phantom 13 corresponding one of the imaging conditions 22 different from each other and generating the plurality of correction curves 20a by repeating steps 101 to 104.

Also, each of the plurality of correction curve sets 20 is generated in corresponding one of the plurality of types of correction phantoms 13 in steps for capturing an image of the corresponding one of the plurality of types of correction phantoms 13, which have X-ray absorptances different from each other, depending on materials of the different subjects 90 and generating the plurality of correction curves 20a by repeating steps 101 to 106.

The processing for correcting the subject image 35 by using the X-ray image processing apparatus 6 is now described with reference to FIG. 16.

In step 200, the image acquirer 9 acquires the subject image 35 of the subject 90 captured including the first dark field image 35a (see FIG. 13(A)) and the first absorption image 35b (see FIG. 13(B)).

In step 201, the controller 7b acquires the imaging condition 22. In this embodiment, the controller 7b acquires a value of tube voltage applied to the X-ray source 1 as the imaging condition 22.

In step 202, the controller 7b acquires the material of the subject 90. Specifically, the controller 7b accepts an input of the material of the subject 90. The controller 7b accepts the input of the material of the subject 90 by accepting the operating input 21 through the input acceptor 10.

In step 203, the controller 7b acquires the correction curve set 20 that is used to correct the first dark field image 35a from the plurality types of correction curve sets 20 based on the imaging condition 22 and the material of the subject 90.

In step 204, the controller 7b corrects the subject image 35 (first dark field image 35a) based on the plurality of correction curves 20a. Specifically, the controller 7b corrects the first dark field image 35a based on the first absorption image 35b (see FIG. 13(B)) and the plurality of correction curves 20a. More specifically, the controller 7b corrects the subject image 35 (first dark field image 35a) based on the plurality of correction curves 20a corresponding to the material of the subject 90 accepted as the input in the plurality of correction curve sets 20. The controller 7b corrects the subject image 35 (first dark field image 35a) based on the plurality of correction curves 20a corresponding to the acquired imaging condition 22 in the plurality of correction curve sets 20. In other words, the controller 7b determines the correction curve set 20 to be used for the correction of the subject image 35 from the plurality of correction curve sets 20 based on the material of the subject 90 and the imaging condition 22, and corrects the subject image 35. After that, the procedure ends.

(Advantages of the Embodiment)

In this embodiment, the following advantages are obtained.

In the aforementioned embodiment, the phase contrast X-ray imaging apparatus 100 includes an X-ray source 1; an X-ray detector 2 for detecting the X-rays for irradiation from the X-ray source 1; a plurality of gratings arranged between the X-ray source and the X-ray detector 2; an image processor 7a for generating a phase contrast X-ray image 30 including a first dark field image 35a, which is a dark field image of a subject 90, based on an intensity distribution of the X-rays detected by the X-ray detector 2; a storage 8 for storing a plurality of correction curves 20a generated at positions in a direction perpendicular to a grating direction in which the plurality of gratings extend; and a controller 7b for correcting the first dark field image 35a by using the correction curves 20a corresponding to the positions, wherein each of the correction curves 20a represents an assignment relation between values that relate to X-ray absorption of the subject and values that relate to X-ray scattering of the subject 90 at corresponding one of the positions.

Accordingly, the storage 8 stores a plurality of correction curves 20a corresponding to the positions in a direction (Y direction) perpendicular to the grating direction (Z direction). Because each of the correction curves 20a represents an assignment relation between values that relate to X-ray absorption of the subject 90 and values that relate to X-ray scattering of the subject at corresponding one of the positions, a change degree of a spectrum of X-rays depending on an incident angle of the X-rays at each position is taken into account. Also, the first dark field image 35a can be accurately corrected by correcting the first dark field image 35a, which is a dark field image of a subject 90, by using the correction curves 20a corresponding to the positions, as compared with a case in which the first dark field image 35a is corrected by using a single common correction curve 20a. Consequently, it is possible to reduce deterioration of image quality of a first dark field image 35a even in a case in which X-rays are incident on a grating in an inclined direction. Also, according to the X-ray image processing apparatus 6 and the X-ray image processing method according to the aforementioned embodiment, similar to the phase contrast X-ray imaging apparatus 100 according to the aforementioned embodiment, it is possible to reduce deterioration of image quality of a first dark field image 35a even in a case in which X-rays are incident on a grating in an inclined direction.

Also, in the aforementioned embodiment, the correction curve generation method includes acquiring a second dark field image 31, which is a dark field image of a correction phantom 13, by capturing an image of the correction phantom 13 using a phase contrast X-ray imaging apparatus 100 including a plurality of gratings; acquiring a second absorption image 32, which is an absorption image of the correction phantom 13; and generating the plurality of correction curves 20a at positions in the direction (Y direction) perpendicular to the grating direction (Z direction) in which the plurality of gratings extend by using the second dark field image 31 and the second absorption image 32. Accordingly, because the plurality of correction curves 20a are generated by using the second dark field image 31 and the second absorption image 32 acquired by actually capturing the correction phantom 13, it is possible to generate the plurality of correction curves 20a with a change degree of a spectrum of X-rays depending on an incident angle of the X-rays at each position being highly accurately being reflected.

In addition, following additional advantages can be obtained by the aforementioned embodiment added with configurations discussed below.

In other words, in this embodiment, as described above, the image processor 7a generates a first absorption image 35b of the subject 90, which is an absorption image of the subject 90, in addition to the first dark field image 35a; and the controller 7b is configured to access, for each of the positions, corresponding one of the correction curves 20a, to specify values that relate to the X-ray scattering and are assigned to values that relate to the X-ray absorption in the first absorption image 35b, and to correct the first dark field image 35a by using the specified values, which relate to the X-ray scattering. In addition, the controller 7b acquires values that relate to the X-ray absorption at the aforementioned positions in the first absorption image 35b. The controller 7b accesses, for each of the positions, corresponding one of the correction curves 20a, and acquires values that relate to X-ray scattering corresponding to the values that relate to X-ray absorption. Subsequently, the controller 7b corrects the first dark field image 35a by using the X-ray scattering values acquired. As a result, the first dark field image 35a can be accurately corrected by executing these processes as described above.

In this embodiment, as described above, the storage 8 is configured to store the plurality of correction curves 20a to associate the plurality of correction curves to materials of subjects 90; and the controller 7b is configured to correct the first dark field image 35a by selecting, in accordance with a material of the subject 90, corresponding ones of the plurality of correction curves 20a, and by using the selected ones of the plurality of correction curves 20a. Accordingly, because the correction curves 20a that correspond to the material of the subject 90 is selected, the first dark field image 35a can be corrected by the correction curve 20a that correspond to the X-ray absorptance of the subject 90. Consequently, reduction of accuracy of correction of the first dark field image 35a is suppressed as compared with, for example, a configuration in which the first dark field image 35a is corrected by using one type of correction curve 20a independent of the material of the subject 90, it is possible to further reduce deterioration of image quality of the first dark field image 35a.

In this embodiment, as described above, the storage 8 is configured to store the plurality of correction curves 20a to associate the plurality of correction curves to imaging conditions 22; and the controller 7b is configured to correct the first dark field image 35a by selecting, in accordance with one of the imaging conditions 22, corresponding ones of the plurality of correction curves 20a, and by using the selected ones of the plurality of correction curves 20a. Here, when setting of the imaging condition 22 is changed, a spectrum of X-rays detected by the X-ray detector 2 is changed. For this reason, in a case in which one type of correction curve 20a is used to correct the first dark field image 35a independent of the imaging condition 22, accuracy of the correction may be reduced. To address this, the plurality of correction curves 20a that correspond to the imaging condition 22 set is used to correct the first dark field image 35a in the aforementioned configuration, and as a result it is possible to suppress reduction of correction accuracy of the first dark field image 35a.

[Modified Embodiments]

Note that the embodiment disclosed this time must be considered as illustrative in all points and not restrictive. The scope of the present invention is not shown by the above description of the embodiments but by the scope of claims for patent, and all modifications (modified embodiments) within the meaning and scope equivalent to the scope of claims for patent are further included.

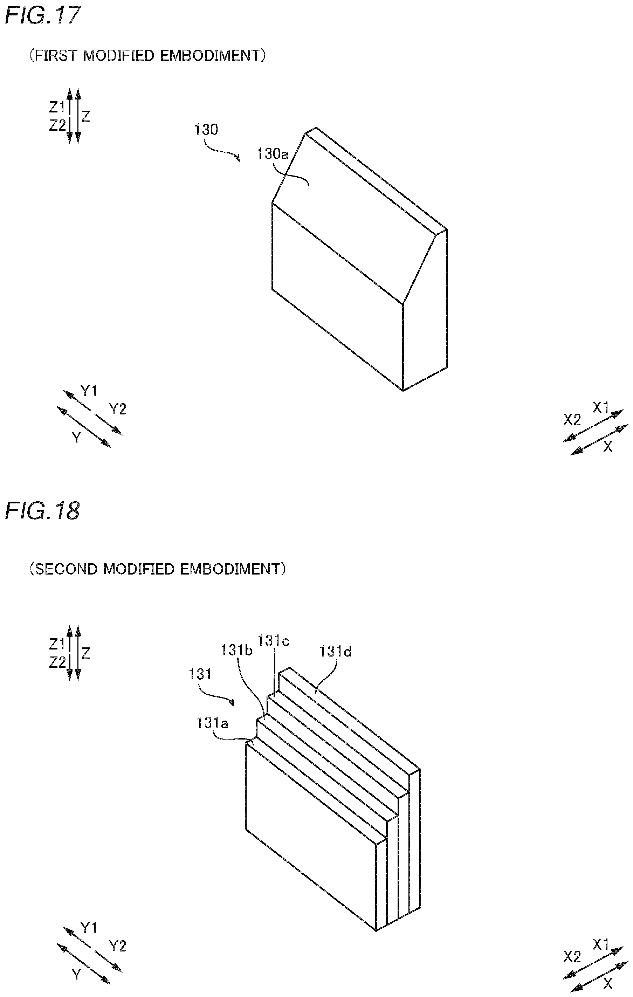

For example, while the example in which the correction phantom 13 has a stepped shape in which its thickness in the X-ray irradiation direction (X direction) changes in steps in the Z direction has been shown in the aforementioned embodiment, the present invention is not limited to this. For example, a thickness in an X-ray irradiation direction (X direction) of the correction phantom may continuously change as in a correction phantom 130 according to a first modified embodiment shown in FIG. 17. Specifically, as shown in FIG. 17, the correction phantom 130 according to the first modified embodiment may have an inclined surface 130a.

While the example in which the correction phantom 13 is integrally formed of a homogeneous material has been shown in the aforementioned embodiment, the present invention is not limited to this. For example, the correction phantom 13 is not necessarily integrally formed. For example, the correction phantom may be constructed of a combination of a plurality types of plate-shaped materials as in a correction phantom 131 according to a second modified embodiment shown in FIG. 18. Specifically, the correction phantom 131 according to the second modified embodiment is constructed of a combination of a first plate-shaped material 131a, a second plate-shaped material 131b, a third plate-shaped material 131c, and a fourth plate-shaped material 131d.

While the example in which the phase contrast X-ray imaging apparatus 100 captures an image with orientations of the gratings being fixed has been shown in the aforementioned embodiment, the present invention is not limited to this. For example, the phase contrast X-ray imaging apparatus may include a grating rotation mechanism for rotating the plurality of gratings about an optical axis of X-rays, and may be configured to capture images while changing angles of the plurality of gratings at different.

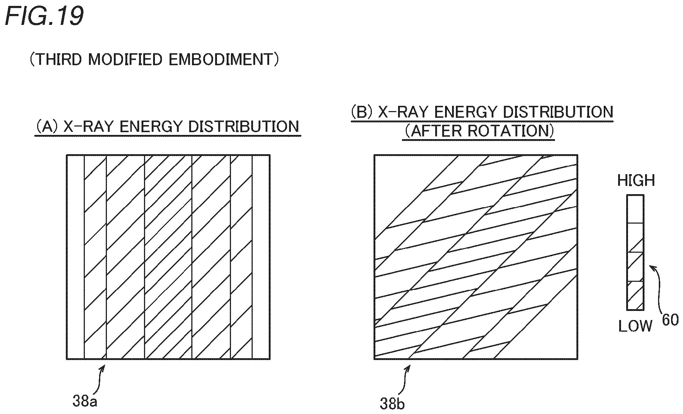

When the plurality of gratings are rotated, an energy distribution of X-rays detected by the X-ray detector 2 changes. Difference between energy distributions of X-rays due to difference between different rotation angles of the plurality of gratings is now described with reference to FIGS. 19(A) and 19(B). Here, the difference between energy distributions of X-rays is represented by different hatching patterns indicated by legends 60 in illustrations of FIGS. 19(A) and 19(B). Specifically, the denser the hatching pattern (the narrower the distance between hatching lines), the lower the energy of the X-rays. Note that, although boundary lines are shown between areas of different X-ray energies, the energy of X-rays actually smoothly changes.

An illustration of FIG. 19(A) shows an image 38a of an energy distribution of X-rays on the X-ray detector 2 when the plurality of gratings are orientated with their grating directions agreeing with the Z direction. In the illustration shown in FIG. 19(A), because the grating directions agree with the Z direction, the energy distribution of the X-rays changes in the Y direction, which is perpendicular to the grating direction. Specifically, in the Y-direction, the energy of the X-rays in the center of the image 38a is low, and the energy of the X-rays increases toward the edge of the image 38a.

An illustration of FIG. 19(B) shows an image 38b of an energy distribution of X-rays when the plurality of gratings are rotated about an axis of an irradiation axis direction of the X-rays (X direction). The image 38b shown in FIG. 19(B) is an image of an energy distribution of X-rays when the plurality of gratings are rotated 45 degrees. When the plurality of gratings are rotated, the energy distribution of the X-rays correspondingly rotates in accordance with a direction of the X-rays. In other words, the image 38b is an image corresponding to the image 38a of the energy distribution that is rotated 45 degrees.

For this reason, even when the plurality of gratings are rotated about the irradiation axis of the X-rays, it is not necessary to capture a correction image of a correction phantom 13 (see FIG. 8) that is rotated at the same angle as the gratings to use the correction image to correct the first dark field image 35a (see FIG. 14(A)) because the first dark field image can be corrected by applying a coordinate transformation to the correction curves 20a previously stored in the storage 8 (see FIG. 1). Accordingly, the controller 7b corrects the first dark field image 35a by converting coordinates of the plurality of correction curves 20a.

While the example in which the controller 7b corrects the first dark field image 35a by using the plurality of correction curves 20a that correspond to a material of the subject 90 in the plurality types of correction curve sets 20, which are generated corresponding to materials of the subjects 90 has been shown in the aforementioned embodiment, the present invention is not limited to this. For example, the controller may be configured to correct the first dark field image 35a by using the plurality of correction curves 20a included in one type of correction curve set independent of the material of the subject 90. However, if the controller corrects the first dark field image 35a by using the plurality of correction curves 20a included in one type of correction curve set independent of the material of the subject 90, accuracy of the correction decreases. For this reason, the controller is preferably configured to correct the first dark field image 35a by using the plurality of correction curves 20a included in one type of correction curve set.

Also, in the aforementioned embodiment, the phase contrast X-ray imaging apparatus 100 does not necessarily include the input acceptor 10. That is, the controller 7b may automatically select the plurality of correction curves 20a by using the first dark field image 35a and the first absorption image 35b. For example, it can be conceived that techniques such as machine learning or pixel processing may be applied to the selection.

While the example in which the storage 8 stores the plurality of correction curve sets 20, which are generated based on the correction images 33 captured under imaging conditions 22 different from each other, for the imaging conditions 22, and the controller 7b corrects the first dark field image 35a based on the plurality of correction curves 20a that correspond to the acquired imaging condition 22 has been shown in the aforementioned embodiment, the present invention is not limited to this. For example, the storage 8 does not necessarily store the plurality of correction curve sets 20 for the imaging conditions 22. In this case, the controller 7b can correct the first dark field image 35a by using one type of correction curve set 20 independent of the imaging condition 22. However, if the storage 8 does not store the plurality of correction curve sets 20 for the imaging conditions 22, accuracy of correction of the first dark field image 35a may decrease in a case in which the imaging condition 22 is changed. For this reason, the storage 8 preferably stores the plurality of correction curve sets 20 for the imaging conditions 22.

While the example in which the X-ray image processing apparatus 6 generates the correction curves 20a has been shown in the aforementioned embodiment, the present invention is not limited to this. For example, the X-ray image processing apparatus 6 may be configured to store the plurality of correction curves 20a, which are generated by another image processing apparatus.

While the example in which the X-ray image processing apparatus 6 generates the plurality of correction curves 20a by using the correction image 33 of the correction phantom 13, which is captured with the correction phantom being orientated to make a direction in which a thickness of the correction phantom 13 agree with the grating direction (Z direction) has been shown in the aforementioned embodiment, the present invention is not limited to this. When the correction phantom 13 is placed, the direction in which the thickness of the correction phantom 13 changes does not necessarily agree with the grating direction. However, if the direction in which the thickness of the correction phantom 13 changes does not agree with the grating direction, it is difficult to compensate for uneven noise caused by X-rays incident on the grating in an inclined direction. For example, when the correction image 33 is captured, it is preferable to make the direction in which the thickness of the correction phantom 13 changes agree with the grating direction.

While the example in which when the controller 7b uses one type of correction curve set 20 to correct the first dark field image 35a has been shown in the aforementioned embodiment, the present invention is not limited to this. For example, in a case in which the controller corrects a dark field image of a subject that includes different materials stacked in the illumination direction of X-rays, the controller may use a plurality of correction curve sets 20 to correct the dark field image. Specifically, in a case in which the controller corrects a subject that includes an acrylic material and an aluminum material stacked in the illumination direction of X-rays, the controller may use the correction curves 20a that are suitable for the acrylic materials and the correction curves 20a that are suitable for the aluminum material together. In the case in which the controller uses the correction curves 20a that are suitable for the acrylic materials and the correction curves 20a that are suitable for the aluminum material together, the controller can use the correction curves that are obtained by averaging the correction curves 20a that are suitable for the acrylic materials and the correction curves 20a that are suitable for the aluminum material to correct the dark field image.

While the example in which the phase contrast X-ray imaging apparatus 100 includes the X-ray image processing apparatus 6 has been shown in the aforementioned embodiment, the present invention is not limited to this. For example, the phase contrast X-ray imaging apparatus 100 does not necessarily include the X-ray image processing apparatus 6. The X-ray image processing apparatus 6 may be provided independently of the phase contrast X-ray imaging apparatus 100. In this case, the first dark field image 35a can be corrected by processing the phase contrast X-ray image 30, which is acquired by the phase contrast X-ray imaging apparatus 100, by using the X-ray image processing apparatus 6.

While the example in which the grating position adjustment mechanism 12 adjusts a position of the second grating 4 has been shown in the aforementioned embodiment, the present invention is not limited to this. The grating position adjustment mechanism 12 may be configured to adjust a position of a grating other than the second grating 4. The grating position adjustment mechanism 12 can adjust a position of any of the plurality of gratings.

[Modes]

The aforementioned exemplary embodiment will be understood as concrete examples of the following modes by those skilled in the art.

(Mode Item 1)

A phase contrast X-ray imaging apparatus includes an X-ray source; an X-ray detector for detecting the X-rays for irradiation from the X-ray source; a plurality of gratings arranged between the X-ray source and the X-ray detector; an image processor for generating a phase contrast X-ray image including a first dark field image, which is a dark field image of a subject, based on an intensity distribution of the X-rays detected by the X-ray detector; a storage for storing a plurality of correction curves generated at positions in a direction perpendicular to a grating direction in which the plurality of gratings extend; and a controller for correcting the first dark field image by using the correction curves corresponding to the positions, wherein each of the plurality of correction curves represents an assignment relation between values that relate to X-ray absorption of the subject and values that relate to X-ray scattering of the subject at corresponding one of the positions.

(Mode Item 2)

In the phase contrast X-ray imaging apparatus according to mode item 1, the image processor generates a first absorption image of the subject, which is an absorption image of the subject, in addition to the first dark field image; and the controller is configured to access, for each of the positions, corresponding one of the correction curves, to specify values that relate to the X-ray scattering and are assigned to values that relate to the X-ray absorption in the first absorption image, and to correct the first dark field image by using the specified values, which relate to the X-ray scattering.

(Mode Item 3)

In the phase contrast X-ray imaging apparatus according to mode item 1 or 2, the storage is configured to store the plurality of correction curves to associate the plurality of correction curves to materials of subjects; and the controller is configured to correct the first dark field image by selecting, in accordance with a material of the subject, corresponding ones of the plurality of correction curves, and by using the selected ones of the plurality of correction curves.

(Mode Item 4)

In the phase contrast X-ray imaging apparatus according to mode item 1 or 2, the storage is configured to store the plurality of correction curves to associate the plurality of correction curves to imaging conditions; and the controller is configured to correct the first dark field image by selecting, in accordance with one of the imaging conditions, corresponding ones of the plurality of correction curves, and by using the selected ones of the correction curves.

(Mode Item 5)

An X-ray image processing apparatus includes an image acquirer for acquiring a phase contrast X-ray image including a first dark field image, which is a dark field image of a subject; a storage for storing a plurality of correction curves generated at positions in a direction perpendicular to a grating direction in which a plurality of gratings extend; and a controller for correcting the first dark field image by using the correction curves corresponding to the positions, wherein each of the plurality of correction curves represents an assignment relation between values that relate to X-ray absorption of the subject and values that relate to X-ray scattering of the subject at corresponding one of the positions.

(Mode Item 6)

An X-ray image processing method includes acquiring a phase contrast X-ray image including a first dark field image, which is a dark field image of a subject; storing a plurality of correction curves generated at positions in a direction perpendicular to a grating direction in which a plurality of gratings extend; and correcting the first dark field image by using the correction curves corresponding to the positions, wherein each of the plurality of correction curves represents an assignment relation between values that relate to X-ray absorption of the subject and values that relate to X-ray scattering of the subject at corresponding one of the positions.

(Mode Item 7)

A method for generating the plurality of correction curves to be used in the X-ray image processing method according to mode item 6, the correction curve generation method includes acquiring a second dark field image, which is a dark field image of a correction phantom, by capturing an image of the correction phantom by using a phase contrast X-ray imaging apparatus including a plurality of gratings; acquiring a second absorption image, which is an absorption image of the correction phantom; and generating the plurality of correction curves at positions in the direction perpendicular to the grating direction in which the plurality of gratings extend by using the second dark field image and the second absorption image.

(Mode Item 8)

In the correction curve generation method according to mode item 7, the correction phantom has a structure whose X-ray absorption degree changes in the grating direction of the plurality of gratings; and in the generating the plurality of correction curves, each of the plurality of correction curves is generated by acquiring an assignment relation between values that relate to the X-ray absorption and are specified based on the second absorption image, and values that relate to the X-ray scattering and are specified based on the second dark field image at corresponding one of the positions.

(Mode Item 9)

In the correction curve generation method according to mode item 7, the correction phantom has a structure whose thickness in an X-ray irradiation direction changes in the grating direction of the plurality of gratings; and in the generating the plurality of correction curves, each of the plurality of correction curves is generated by acquiring an assignment relation between values that relate to the X-ray absorption and are specified based on the second absorption image, and values that relate to the X-ray scattering and are specified based on the second dark field image at corresponding one of the positions. Description of Reference Numerals
1; X-ray source
2; X-ray detector
3; first grating (one of gratings)
4; second grating (one of gratings)
5; third grating (one of gratings)

6; X-ray image processing apparatus
7a; image processor
7b; controller
8; storage
9; image acquirer
13, 13e, 13f, 130, 131; correction phantom
22; imaging condition
30; phase contrast X-ray image
31; second dark field image (correction phantom dark field image)
32; second absorption image (absorption image of correction phantom)
35a; first dark field image (dark field image of subject)
35b; first absorption image (absorption image of subject)
90; subject
100; phase contrast X-ray imaging apparatus

## Claims

1. A phase contrast X-ray imaging apparatus comprising:

   an X-ray source;
   an X-ray detector for detecting the X-rays for irradiation from the X-ray source;
   a plurality of gratings arranged between the X-ray source and the X-ray detector;
   an image processor for generating a phase contrast X-ray image including a first dark field image, which is a dark field image of a subject, based on an intensity distribution of the X-rays detected by the X-ray detector;
   a storage for storing a plurality of correction curves generated at positions in a direction perpendicular to a grating direction in which the plurality of gratings extend; and
   a controller for correcting the first dark field image by using the correction curves corresponding to the positions, wherein
   each of the plurality of correction curves represents an assignment relation between values that relate to X-ray absorption of the subject and values that relate to X-ray scattering of the subject at corresponding one of the positions.

2. The phase contrast X-ray imaging apparatus according to claim 1, wherein

   the image processor generates a first absorption image of the subject, which is an absorption image of the subject, in addition to the first dark field image; and
   the controller is configured to access, for each of the positions, corresponding one of the correction curves, to specify values that relate to the X-ray scattering and are assigned to values that relate to the X-ray absorption in the first absorption image, and to correct the first dark field image by using the specified values, which relate to the X-ray scattering.

3. The phase contrast X-ray imaging apparatus according to claim 1 or 2, wherein

   the storage is configured to store the plurality of correction curves to associate the plurality of correction curves to materials of subjects; and
   the controller is configured to correct the first dark field image by selecting, in accordance with a material of the subject, corresponding ones of the plurality of correction curves, and by using the selected ones of the plurality of correction curves.

4. The phase contrast X-ray imaging apparatus according to claim 1 or 2, wherein

   the storage is configured to store the plurality of correction curves to associate the plurality of correction curves to imaging conditions; and
   the controller is configured to correct the first dark field image by selecting, in accordance with one of the imaging conditions, corresponding ones of the plurality of correction curves, and by using the selected ones of the plurality of correction curves.

5. An X-ray image processing apparatus comprising:

   an image acquirer for acquiring a phase contrast X-ray image including a first dark field image, which is a dark field image of a subject;
   a storage for storing a plurality of correction curves generated at positions in a direction perpendicular to a grating direction in which a plurality of gratings extend; and
   a controller for correcting the first dark field image by using the correction curves corresponding to the positions, wherein
   each of the plurality of correction curves represents an assignment relation between values that relate to X-ray absorption of the subject and values that relate to X-ray scattering of the subject at corresponding one of the positions.

6. An X-ray image processing method comprising:

   acquiring a phase contrast X-ray image including a first dark field image, which is a dark field image of a subject;
   storing a plurality of correction curves generated at positions in a direction perpendicular to a grating direction in which a plurality of gratings extend; and

correcting the first dark field image by using the correction curves corresponding to the positions, wherein each of the plurality of correction curves represents an assignment relation between values that relate to X-ray absorption of the subject and values that relate to X-ray scattering of the subject at corresponding one of the positions.

7. A method for generating the plurality of correction curves to be used in the X-ray image processing method according to claim 6, the correction curve generation method comprising:

acquiring a second dark field image, which is a dark field image of a correction phantom, by capturing an image of the correction phantom by using a phase contrast X-ray imaging apparatus including a plurality of gratings; acquiring a second absorption image, which is an absorption image of the correction phantom; and generating the plurality of correction curves at positions in the direction perpendicular to the grating direction in which the plurality of gratings extend by using the second dark field image and the second absorption image.

8. The correction curve generation method according to claim 7, wherein

the correction phantom has a structure whose X-ray absorption degree changes in the grating direction of the plurality of gratings; and in the generating the plurality of correction curves, each of the plurality of correction curves is generated by acquiring an assignment relation between values that relate to the X-ray absorption and are specified based on the second absorption image, and values that relate to the X-ray scattering and are specified based on the second dark field image at corresponding one of the positions.

9. The correction curve generation method according to claim 7, wherein

the correction phantom has a structure whose thickness in an X-ray irradiation direction changes in the grating direction of the plurality of gratings; and in the generating the plurality of correction curves, each of the plurality of correction curves is generated by acquiring an assignment relation between values that relate to the X-ray absorption and are specified based on the second absorption image, and values that relate to the X-ray scattering and are specified based on the second dark field image at corresponding one of the positions.

*FIG.1*

PHASE CONTRAST X-RAY IMAGE — 30

X-RAY IMAGE PROCESSING APPARATUS — 6

PROCESSOR — 7
IMAGE PROCESSOR — 7a
CONTROLLER — 7b

IMAGE ACQUIRER — 9

STORAGE — 8
CORRECTION CURVE SET — 20
CORRECTION CURVE — 20a

IMAGING CONDITION — 22

GRATING POSITION ADJUSTMENT MECHANISM — 12

OPERATING INPUT — 21

DISPLAY — 11

INPUT ACCEPTOR — 10

*FIG.2*

*FIG.3*

ABSORPTION IMAGE : $\dfrac{Cs}{Cr}$ (INTENSITY RATIO)

PHASE DIFFERENTIAL IMAGE : $const \cdot \Delta\phi$ (PHASE CHANGE MAGNITUDE)

DARK FIELD IMAGE : $\dfrac{Vs}{Vr} = \dfrac{As/Cs}{Ar/Cr}$ (SCATTERING DEGREE)

FIG.4

31  90  SECOND  91
DARK FIELD IMAGE

40

90b  90a  90c

Y

FIG.5

50

50a

PIXEL VALUE

POSITION (PIXEL)

## FIG.6

## FIG.7

FIG.8

FIG.9

## FIG.10

(A) SECOND
DARK FIELD IMAGE

(B) SECOND
ABSORPTION IMAGE

## FIG.11

ABSORPTION VALUE

DARK FIELD IMAGE VALUE

*FIG.12*

# FIG.13

(A) SUBJECT IMAGE
(FIRST DARK FIELD IMAGE)

(B) SUBJECT IMAGE
(FIRST ABSORPTION IMAGE)

(C) CORRECTION CURVE    53

DARK FIELD IMAGE VALUE

*FIG.14*

(A) SUBJECT IMAGE
(BEFORE CORRECTION)

41a
41b
41c
41d
41e

74a
74b
74c
74d
74e

35a

(B) PIXEL VALUE
(BEFORE CORRECTION)

PIXEL VALUE

54
54a
54b
54c
54d
54e

POSITION (PIXEL)

(C) SUBJECT IMAGE AFTER
CORRECTION (COMP. ex)

42a
42b
42c
42d
42e

74a
74b
74c
74d
74e

35c

(D) PIXEL VALUE AFTER
CORRECTION (COMP. ex)

PIXEL VALUE

55
55a
55b
55c
55d
55e

POSITION (PIXEL)

(E) SUBJECT IMAGE AFTER
CORRECTION (EMBODIMENT)

43a
43b
43c
43d
43e

74a
74b
74c
74d
74e

35d

(F) PIXEL VALUE AFTER
CORRECTION (EMBODIMENT)

PIXEL VALUE

56
56a
56b
56c
56d
56e

POSITION (PIXEL)

*FIG.15*

CORRECTION CURVE
GENERATION PROCESSING

( START )

CAPTURE
CORRECTION IMAGE  ⌐101

GENERATE
CORRECTION CURVES  ⌐102

CHANGE IMAGING CONDITION  104

GENERATE
CORRECTION CURVES
UNDER ALL IMAGING
CONDITIONS
?  103

NO

YES

CHANGE
CORRECTION PHANTOM  106

GENERATE
CORRECTION CURVES
FOR ALL CORRECTION
PHANTOMS
?  105

NO

YES

( END )

## FIG.16

SUBJECT IMAGE
CORRECTION PROCESSING

START

ACQUIRE SUBJECT IMAGE — 200

ACQUIRE
IMAGING CONDITION — 201

ACQUIRE
MATERIAL OF SUBJECT — 202

ACQUIRE
CORRECTION CURVES — 203

CORRECT SUBJECT IMAGE — 204

END

FIG.17

(FIRST MODIFIED EMBODIMENT)

Z1
Z2 Z

130
130a

Y1
Y2
Y

X2 X1
X

FIG.18

(SECOND MODIFIED EMBODIMENT)

Z1
Z2 Z

131b 131c
131 131d
131a

Y1
Y2
Y

X2 X1
X

# FIG.19

(THIRD MODIFIED EMBODIMENT)

(A) X-RAY ENERGY DISTRIBUTION

(B) X-RAY ENERGY DISTRIBUTION
(AFTER ROTATION)

HIGH

LOW 60

38a

38b

## INTERNATIONAL SEARCH REPORT

| International application No. |
|---|
| **PCT/JP2023/017327** |

**A. CLASSIFICATION OF SUBJECT MATTER**

***G01N 23/041***(2018.01)i; ***G01N 23/083***(2018.01)i
FI: G01N23/041; G01N23/083

According to International Patent Classification (IPC) or to both national classification and IPC

**B. FIELDS SEARCHED**

Minimum documentation searched (classification system followed by classification symbols)

G01N23/041; G01N23/083

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII), JSTChina (JDreamIII), Science Direct, PubMed

**C. DOCUMENTS CONSIDERED TO BE RELEVANT**

| Category* | Citation of document, with indication, where appropriate, of the relevant passages | Relevant to claim No. |
|---|---|---|
| A | JP 2019-072367 A (SHIMADZU CORP) 16 May 2019 (2019-05-16)<br>claim 1, paragraphs [0010]-[0024] | 1-9 |
| A | WO 2019/073760 A1 (SHIMADZU CORP) 18 April 2019 (2019-04-18)<br>fig. 1-11 | 1-9 |
| A | WO 2011/052419 A1 (HITACHI, LTD.) 05 May 2011 (2011-05-05)<br>paragraph [0103] | 1-9 |
| A | JP 2017-205138 A (KONICA MINOLTA INC) 24 November 2017 (2017-11-24)<br>fig. 7 | 1-9 |
| A | JP 2020-527989 A (KONINKLIJKE PHILIPS N.V.) 17 September 2020 (2020-09-17) | 1-9 |
| A | JP 2014-239873 A (CANON KK) 25 December 2014 (2014-12-25) | 1-9 |
| A | JP 2012-090944 A (FUJIFILM CORP) 17 May 2012 (2012-05-17) | 1-9 |
| A | JP 2017-516558 A (KONINKLIJKE PHILIPS N.V.) 22 June 2017 (2017-06-22) | 1-9 |

☐ Further documents are listed in the continuation of Box C.      ☑ See patent family annex.

| | |
|---|---|
| \* Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **08 June 2023** | **20 June 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT
### Information on patent family members

International application No.

**PCT/JP2023/017327**

| Patent document cited in search report | | | Publication date (day/month/year) | Patent family member(s) | | | Publication date (day/month/year) |
|---|---|---|---|---|---|---|---|
| JP | 2019-072367 | A | 16 May 2019 | (Family: none) | | | |
| WO | 2019/073760 | A1 | 18 April 2019 | US fig. 1-11 CN | 2020/0196969 110913764 | A1 A | |
| WO | 2011/052419 | A1 | 05 May 2011 | (Family: none) | | | |
| JP | 2017-205138 | A | 24 November 2017 | US fig. 7 | 2017/0325765 | A1 | |
| JP | 2020-527989 | A | 17 September 2020 | US WO EP EP CN | 2020/0205765 2019/020748 3435325 3659107 110998653 | A1 A1 A1 A1 A | |
| JP | 2014-239873 | A | 25 December 2014 | US EP | 2014/0341335 2804148 | A1 A2 | |
| JP | 2012-090944 | A | 17 May 2012 | US | 2011/0243305 | A1 | |
| JP | 2017-516558 | A | 22 June 2017 | US WO EP CN RU | 2017/0122885 2015/180977 3149459 106413556 2016150815 | A1 A1 A1 A A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021194389 A